# EUROPÄISCHE PATENTSCHRIFT

(11) **EP 3 583 215 B1**
(45) Veröffentlichungstag und Bekanntmachung des Hinweises auf die Patenterteilung: **19.03.2025**
(21) Anmeldenummer: 18710763.6
(22) Anmeldetag: 09.02.2018
(51) Int. Cl.: C12N 15/113, C12N 15/87, C12N 15/67

(54) **SYSTEM UND VERFAHREN ZUR ZELLTYP-SPEZIFISCHEN TRANSLATION VON RNA-MOLEKÜLEN IN EUKARYOTEN**
SYSTEM AND PROCESS FOR CELL-TYPE SPECIFIC TRANSLATION OF RNA MOLECULES IN EUKARYOTES
SYSTÈME ET PROCÉDÉ POUR LA TRADUCTION SPÉCIFIQUE DE TYPE CELLULAIRE DE MOLÉCULES D'ARN DANS DES EUCARYOTES

(30) Priorität: 20.02.2017 DE 102017103383
(43) Veröffentlichungstag der Anmeldung: 25.12.2019
(73) Patentinhaber: Arena-Bio Gbr, 50733 Köln (DE)
(72) Erfinder: BOHLEN, Heribert, 50733 Köln (DE); HOFFMANN, Bernd, 52428 Jülich (DE)
(74) Vertreter: Kuttenkeuler, David
(86) Internationale Anmeldenummer: PCT/EP2018/053241
(87) Internationale Veröffentlichungsnummer: WO 2018/149740

(56) Entgegenhaltungen:
- WO-A2-2015/042184
- J. R. BABENDURE ET AL: "Control of mammalian translation by mRNA structure near caps", RNA, vol. 12, no. 5, 1 January 2006 (2006-01-01), US, pages 851 - 861, XP055470459, ISSN: 1355-8382, DOI: 10.1261/rna.2309906
- MOHAMMAD ALI FAGHIHI ET AL: "Regulatory roles of natural antisense transcripts", NATURE REVIEWS MOLECULAR CELL BIOLOGY, vol. 10, no. 9, 1 September 2009 (2009-09-01), pages 637 - 643, XP055152670, ISSN: 1471-0072, DOI: 10.1038/nrm2738
- MUNROE S H ET AL: "Overlapping transcripts, double-stranded RNA and antisense regulation: A genomic perspective", CMLS CELLULAR AND MOLECULAR LIFE SCIENCES, BIRKHÄUSER-VERLAG, BA, vol. 63, no. 18, 17 July 2006 (2006-07-17), pages 2102 - 2118, XP019440746, ISSN: 1420-9071, DOI: 10.1007/S00018-006-6070-2
- FLORE SINTUREL ET AL: "Cytoplasmic Control of Sense-Antisense mRNA Pairs", CELL REPORTS, vol. 12, no. 11, 1 September 2015 (2015-09-01), US, pages 1853 - 1864, XP055469583, ISSN: 2211-1247, DOI: 10.1016/j.celrep.2015.08.016
- VICENT PELECHANO ET AL: "Gene regulation by antisense transcription", NATURE REVIEWS GENETICS, vol. 14, no. 12, 12 November 2013 (2013-11-12), GB, pages 880 - 893, XP055243155, ISSN: 1471-0056, DOI: 10.1038/nrg3594
- MADHUR KUMAR ET AL: "Antisense RNA: Function and Fate of Duplex RNA in Cells of Higher Eukaryotes", MICROBIOLOGY AND MOLECULAR BIOLOGY REVIEW, vol. 62, no. 4, 1 December 1998 (1998-12-01), pages 1415 - 1434, XP055469597, Retrieved from the Internet <URL:https://www.ncbi.nlm.nih.gov/pmc/articles/PMC98951/pdf/mr001415.pdf>
- A. OGAWA: "Rational design of artificial riboswitches based on ligand-dependent modulation of internal ribosome entry in wheat germ extract and their applications as label-free biosensors", RNA, vol. 17, no. 3, 11 January 2011 (2011-01-11), US, pages 478 - 488, XP055470610, ISSN: 1355-8382, DOI: 10.1261/rna.2433111
- ALEXANDER SERGANOV ET AL: "A Decade of Riboswitches", CELL, vol. 152, no. 1-2, 1 January 2013 (2013-01-01), pages 17 - 24, XP055078909, ISSN: 0092-8674, DOI: 10.1016/j.cell.2012.12.024
- N. STANDART ET AL: "MicroRNAs repress translation of m7Gppp-capped target mRNAs in vitro by inhibiting initiation and promoting deadenylation", GENES AND DEVELOPMENT., vol. 21, no. 16, 15 August 2007 (2007-08-15), US, pages 1975 - 1982, XP055470381, ISSN: 0890-9369, DOI: 10.1101/gad.1591507
- GUNTER MEISTER ET AL: "Mechanisms of gene silencing by double-stranded RNA", NATURE, vol. 431, no. 7006, 16 September 2004 (2004-09-16), pages 343 - 349, XP055153799, ISSN: 0028-0836, DOI: 10.1038/nature02873

## Beschreibung

Die vorliegende Erfindung betrifft Verfahren und RNA-Konstrukte zur zielgerichteten Translation eines Polypeptids von Interesse in einer eukaryotischen Ziel-Zelle, sowie deren Verwendung in therapeutisch klinischen Anwendungen.

### Hintergrund der Erfindung

Die gerichtete Einbringung bestimmter Moleküle, beispielsweise medizinisch und pharmakologisch relevanter Proteine oder die Zellvitalität beeinflussender Peptide in der Krebstherapie stellen eine der größten Notwendigkeiten gleichzeitig aber auch eine bisher noch nicht ansatzweise zufriedenstellend gelöste Schwierigkeit in der modernen Zellbiologie dar.

Unterschiedliche Techniken werden bisher eingesetzt, um Moleküle von Interesse zielgerichtet in komplexen Organismen wie dem Menschen freizusetzen bzw. zu aktivieren. Zwei unterschiedliche Ansätze sind hierbei von besonderem Interesse.

Basierend auf Trägersystemen wie beispielsweise Liposomen (D. Papahadjopoulos, M. Moscarello, E. H. Eylar, T. Isac, Biochim. Biophys. Acta. 1975, 401, 317) werden Moleküle (z.B. Krebs-Therapeutika) an oder in diese Trägersysteme gekoppelt bzw. eingebettet. Nachfolgend werden die hierdurch funktionalisierten Trägersysteme in den Körper eingebracht wobei die Einbringung entweder den gesamten Körper oder nur bestimmte Teile des Organismus umfassen kann. Die Trägersysteme sind hierbei im Idealfall so beschaffen, dass natürliche Körperbedingungen die Stabilität zwischen Trägersystem und Molekül von Interesse nicht beeinflussen und so die Freisetzung des Moleküls unterbleibt. Durch äußere Faktoren können nun die Trägersysteme destabilisiert werden und dadurch die Freisetzung und damit Aktivität der eingebauten Moleküle induzieren. Als äußere Faktoren zur Freisetzung wird speziell mit lokaler Erwärmung bestimmter Körperregionen (Hyperthermieansatz) als auch mit Licht- bzw. pH-induzierter Spaltung spezifischer Bindungen zwischen Trägersystem und Molekül gearbeitet. Zusammenfassend sind all diese Systeme durch Qiu and Park (Advanced Drug Delivery Reviews 53 (2001) 321-339 Environment-sensitive hydrogels for drug delivery) sowie Shamay und Kollegen (Biomaterials, Light induced drug delivery into cancer cells 2011 32:1377-86) vom Prinzip her dargestellt. Problematisch bei all diesen Methoden ist jedoch im Besonderen, dass die Funktionsmoleküle in hoher Konzentration in den gesamten Körper eingebracht werden müssen und es dazu häufig zu Nebenwirkungen und unspezifischen Freisetzungen kommen kann. Hinzu kommt, dass die Methodiken orts- jedoch nicht zelltyp-spezifisch wirken und dadurch beispielsweise Zytostatika neben den vorgesehenen Krebszellen auch umgebendes Gewebe schädigen bzw. auch schnell vom Ort der Freisetzung im Körper verteilt werden. Auch hier muss daher mit hohen Dosen gearbeitet werden.

Als zweite Methodik zur zielgerichteten Freisetzung von Molekülen werden meist Systeme eingesetzt, bei denen die Moleküle von Interesse entweder direkt oder aber wieder mittels Trägersystemen an Liganden gekoppelt werden, die eine hohe Spezifität zu bestimmten Oberflächenmolekülen definierter Zelltypen aufweisen. Hoch-exprimierte Oberflächenrezeptoren von Krebszellen, wie beispielsweise GPRCs oder Folate-Rezeptoren werden so mittels der Liganden gebunden (Lappano R, Maggiolini M., Nat Rev Drug Discov. 2011, 10:47-60, G protein-coupled receptors: novel targets for drug discovery in cancer; Sudimack J, Lee RJ., Adv Drug Deliv Rev. 2000, 41:147-62. Targeted drug delivery via the folate receptor). Nach Bindung der Liganden an die Oberflächenrezeptoren können gebundene Moleküle entweder direkt ihre Aktivität entfalten (beispielsweise Radiopharmazeutika oder MRI-Kontrastierungsreagenzien) oder aber nach phagozytotischer Aufnahme in die Zellen ihre Funktion entfalten (beispielsweise Chemotherapeutika oder DNA-Vektoren). Auch diese Methodik weist jedoch große Nachteile auf, die zum einen in der erneut relativ hohen Gesamtkonzentration im gesamten Körpersystem liegen, zum anderen aber speziell damit zu tun haben, dass die meisten Oberflächenrezeptoren keine absolute Spezifität für einzelne Zelltypen bzw. Krankheitsbilder aufweisen und dadurch eine Kopplung an derartige Rezeptoren mit erhöhten Nebeneffekten in gesunden Geweben einhergehen kann. Zudem induzieren eine Vielzahl spezifischer Rezeptoren nach Kopplung keine phagozytotische Aufnahme der gebundenen Trägersysteme, so dass diese außerhalb der Zellen verbleiben.

Die allergrößte Anzahl biotechnologisch, pharmakologisch und medizinisch relevanter Moleküle zur gezielten Übertragung in definierte Zelltypen/Gewebe stellen Peptide oder Proteine dar, die typischerweise auf DNA-Ebene kodiert sind. Nach Einbringung dieser DNA-Moleküle werden diese in den Zellkern transportiert und nachfolgend über einen hochkonservierten Mechanismus zuerst in RNA-Moleküle umgeschrieben (Transkription), posttranskriptional modifiziert und dann aus dem Zellkern transportiert, um innerhalb des Zytoplasmas erneut durch einen hochkonservierten Mechanismus (Translation) in Aminosäuresequenzen umbeschrieben zu werden. Sowohl der Transkriptions- als auch der Translationsmechanismus sind in allen eukaryotischen Organismen und Zelltypen vom Grundmechanismus her identisch. Dies bedeutet, dass, wenn auch mit unterschiedlicher Effizienz, jede für ein Peptid kodierende DNA-Sequenz in jeder Zelle das gleiche Primärprodukt erzeugt, welches nachfolgend noch weiter modifiziert werden kann. Der Gesamtprozess aus Transkription und Translation wird Expression genannt. Einige RNA-Moleküle verbleiben untranslatiert und üben eine eigenständige Funktion aus.

Selektive, zielgerichtete Expressionen von DNA-Sequenzen sind möglich, wenn der kodierende Bereich eines DNA-Abschnittes durch zelltyp- bzw. organspezifische Promotor- und Enhancersequenzen sowie Differenzierungs- und Maturierungsgrad abhängig reguliert wird. Derartige Sequenzen regulieren die Bindung des Transkriptionsapparates und bewirken damit dessen Aktivierung nur unter definierten Umgebungsbedingungen. Nachteilig für diese Art der zielgerichteten Expression von DNA-Konstrukten ist jedoch, dass 1) DNA-Konstrukte nur sehr schwer mit hoher Effizienz und homogen in alle Zellen innerhalb eines Organismus bzw. eines Gewebes eingebracht werden können, 2) DNA-Konstrukte für die nachfolgende Transkription immer in den Zellkern transportiert werden müssen und dort mit der natürlichen Erbsubstanz wechselwirken, wodurch Mutationen und nachfolgend Krankheiten (z.B. Krebs) entstehen können und 3) organspezifische Promotorregionen oft sehr groß sind und so nicht geeignet für den Einsatz in von extern eingebrachten DNA-Konstrukten sind.

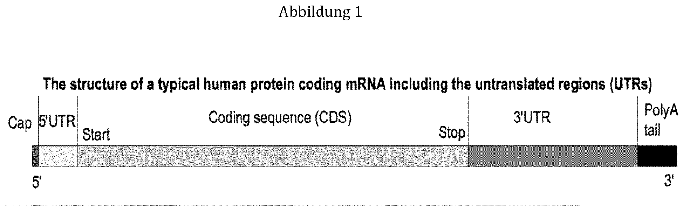

Alternativ zur Verwendung von DNA-Konstrukten zur Expression von Aminosäure-basierten Molekülen kann der Schritt der im Zellkern stattfindenden Transkription umgangen werden, indem direkt RNA Moleküle in Zellen oder Zellgewebe eingeschleust werden. Arbeiten durch unterschiedliche Arbeitsgruppen konnten zeigen (Übersichtsartikel siehe: Van Tendeloo VF, Ponsaerts P, Berneman ZN. mRNA-based gene transfer as a tool for gene and cell therapy. Curr Opin Mol Ther. 2007;9:423-431), dass die Einschleusung von RNA Molekülen hocheffizient und schnell erfolgt ohne dabei signifikante Stresslevel in den Zellen zu erzeugen. Eingebrachte RNA Moleküle verbleiben im Zytoplasma der Zelle und können so nicht mit dem Genom innerhalb des Zellkerns negativ wechselwirken. Durch konservierte Translationsprozesse werden eingebrachte mRNA-Moleküle ebenso wie endogen (natürlich) vorliegende mRNA-Moleküle in Proteine oder Peptide umgewandelt. Hierfür notwendig sind bestimmte Sequenzmotive innerhalb eines mRNA-Moleküls, die in Lehrbüchern (z.B. Alberts et al., Molekularbiologie der Zelle, 2011, Wiley-Blackwell Verlag) detailliert nachgelesen werden können und vereinfacht in Abb. 1 dargestellt sind. Hierbei umfassen mRNA Moleküle typischerweise folgende Einheiten:
1: Cap: Die Cap-Struktur (Kappe) ist eine chemische Veränderung an mRNA-Molekülen in Eukaryoten, die die Stabilität der RNA drastisch erhöht und wichtig für den Transport der RNA aus dem Kern in das Zytoplasma und die darauf folgende Translation der mRNAs durch die Ribosomen ist. Es handelt sich meist um ein modifiziertes Guanin-Nukleotid, welches während der Transkription des Gens über eine seltene 5'-5'-Phosphodiesterbindung an das Kopfende der RNA geknüpft wird.
2: untranslatierter 5'-Bereich: stellt eine, der kodierenden Sequenz vorgeschaltete Nukleotidsequenz dar. Die Region beginnt am Transkriptionsstartpunkt und endet direkt vor dem Translationsstartcodon. Innerhalb dieser Sequenz können regulatorische Sequenzen liegen, die beispielsweise über Sekundärstrukturen die Stabilität der mRNA beeinflussen oder als Bindestellen für Protein fungieren. Außerdem ist typischerweise eine ribosomale Bindestelle vorhanden.
3: kodierende Sequenz: Die kodierende Sequenz beinhaltet die Information für das durch die mRNA herzustellende Protein. Sie beginnt direkt mit dem Translationsstartcodon und endet mit dem Translationsstoppcodon. Die Nukleotidabfolge der kodierenden Sequenz ist durch die Aminosäuresequenz des kodierten Proteins im Rahmen des Triplettcodes vorgegeben. Abhängig vom verwendeten Triplett kann die Expressionsrate als auch die Stabilität der mRNA beeinflusst werden.
4: untranslatierter 3'-Bereich: stellt den Bereich dar, der sich dem für das Protein kodierenden Bereich unmittelbar hinter dem Translationsstoppcodon anschließt. Er umfasst den gesamten Bereich bis hin zum Polyadenylierungsbeginn. Innerhalb des untranslatierten 3'-Bereichs können ebenfalls regulatorische Sequenzen vorhanden sein, die speziell für die Polyadenylierung der RNA als auch für deren Stabilität und Transport von Wichtigkeit sind.
5: polyA-Schwanz: die Polyadenylierung erfolgt durch Anhängen von Adenin-Nukleotiden an das 3'-Ende der mRNA auf Basis einer posttranskriptionellen Modifizierung der mRNA durch die Poly(A)-Polymerase. Der PolyA-Schwanz trägt entscheidend durch seine Länge zur Stabilität der mRNA bei, sowie dadurch dass Proteine an diese Sequenz binden und das CAP mit dem PolyA-Schwanz wechselwirkt. WO2015042184 beschreibt RNA Konstrukte, die nicht kodierende RNA zB. miRNA binden und/oder inhibieren.

Nachteilig auf die Verwendung extern eingebrachter RNA-Moleküle kann sich jedoch für bestimmte Anwendungen auswirken, dass i) RNA-Moleküle typischerweise eine limitierte Lebensdauer aufweisen und dadurch die Bildung von Proteinen nur über einen zeitlich beschränkten Zeitraum erfolgt. Da der grundlegende Mechanismus der Translation weiterhin in allen Eukaryoten und dort existierenden Zelltypen identisch ist, konnte ii) bisher keine zielgerichtete, das heißt Organ- oder Zelltypspezifische Expression von in den Gesamtorganismus eingebrachten mRNA-Moleküle umgesetzt werden.

Neue Forschungsergebnisse (Quabium and Krupp, Synthetic mRNAs for manipulating cellular phenotypes: an overview (2015) New Biotechnology, 32:229-235) ermöglichen inzwischen die sehr effiziente, chemische Modifizierung von mRNA-Molekülen auf unterschiedlichen Ebenen, um dadurch die unter Punkt i) aufgeführte Lebensdauer zu regulieren und wenn nötig deutlich zu erhöhen. Derartige Modifizierung beinhalten die
a) Verwendung chemisch modifizierter Cap-Sequenzen auf Basis des natürlich vorkommenden Caps m7G5'pppN. Beispiele hierfür sind in Jamielity et al. Synthetic mRNA cap analogs with a modified triphosphate bridge - synthesis, applications and prospects (2010) New. J. Chem 34:829-844). Derartige Modifizierungen erhöhen zum einen die Translationseffizienz durch effektive Bindung von Untereinheiten des Translationsapparates. Zum anderen verbessern sie die Stabilität der mRNA Moleküle vermutlich dadurch, dass Nukleasen weniger effizient von Seiten des 5-Endes an die mRNA angreifen können.
b) Nutzung stabiler 5'- und 3'-untranslatierter Bereiche. Typischerweise kann die Stabilität durch Nutzung geeigneter Nukleotidsequenzen deutlich beeinflusst werden. Anlagerung von Bindungspartnern sowie Bildung von Sekundärstrukturen durch Basenpaarung stellen die hauptsächlichen Stabilisierungsgründe dar.
c) Nutzung chemisch modifizierter Nukleotide. Derartige Nukleotide wie beispielsweise 5-methylcytidine oder pseudouridine beeinflussen in erster Linie die Bindung von Endonukleasen und verhindern so den schnellen Abbau der mRNA Moleküle.
d) Verwendung effizienter Polyadenylierungssequenzen im untranslatierten 3'-Bereich, um möglichst lange Polyadenylierungen zu erhalten.

Aufgabe der vorliegenden Erfindung ist es unter anderem Verfahren sowie RNA-Konstrukte bereitzustellen, die eine definierte Expression von mRNA in bestimmten Zelltypen erlaubt.

### Kurzbeschreibung der Erfindung

Ein erster Aspekt der vorliegenden Erfindung bezieht sich auf ein Verfahren zur zielgerichteten Translation eines Polypeptids von Interesse in einer eukaryotischen Ziel-Zelle, gekennzeichnet durch das Einbringen eines RNA-Konstrukts in die Ziel-Zelle, wobei das RNA-Konstrukt in 5' nach 3' Richtung zumindest folgende Elemente aufweist:
(a) ein anticodogenes Element (a), das zumindest zu einem Abschnitt einer mRNA eines durch die Ziel-Zelle exprimierten Gens komplementär ist;
(b) ein blockierendes Element (b), das mindestens mit einem Abschnitt des 3' liegenden Translationsinitiator-Elements (d) wechselwirkt;
(c) ein stabilisierendes Element (c);
(d) ein Translationsinitiator-Element (d), das eine internal ribosomal entry site (IRES) - Sequenz aufweist;
(e) eine für das Polypeptid von Interesse kodierende Sequenz (e),
wobei in einer eukaryotischen Nicht-Ziel-Zelle die Translation des Polypeptids von Interesse durch Basenpaarung des blockierende Elements (b) mit dem Translationsinitiator Element (d) verhindert wird und in einer eukaryotischen Ziel-Zelle die Translation des kodierten Polypeptids aufgrund einer durch die Interaktion des anticodogenen Elements mit der durch die Ziel-Zelle exprimierten mRNA induzierten Prozessierung des RNA-Konstrukts erfolgt.

Ein weiterer Aspekt der vorliegenden Erfindung richtet sich auf ein RNA-Konstrukt zur zielgerichteten Translation eines Polypeptids von Interesse in einer eukaryotischen Ziel-Zelle, wobei das RNA-Konstrukt in 5' nach 3' Richtung zumindest folgende Elemente aufweist:
(a) ein anticodogenes Element (a), das zumindest zu einem Abschnitt einer RNA eines durch die Ziel-Zelle exprimierten DNA-Abschnitts komplementär ist oder den zelltyp-spezifischen Abbau induziert;
(b) ein blockierendes Element (b), das zumindest zu einem Abschnitt des 3' liegenden Translationsinitiator-Elements (d) komplementär ist oder dieses anderweitig inhibiert;
(c) ein stabilisierendes Element (c), das den Ziel-Zell-spezifischen Abbau der RNA auf einen Teilbereich derselben beschränkt;
(d) ein Translationsinitiator-Element (d), das unabhängig vom 5'-Cap funktioniert und nur in Zielzellen aktiv ist; wobei das Translationsinitiator-Element (d) eine internal ribosomal entry site (IRES) - Sequenz aufweist;
(e) eine für das Polypeptid von Interesse kodierende Sequenz (e);
wobei in einer eukaryotischen Nicht-Ziel-Zelle die Translation des Polypeptids von Interesse durch Basenpaarung des blockierenden Elements (b) mit dem Translationsinitiator-Element (d) verhindert wird und in einer eukaryotischen Ziel-Zelle die Translation des kodierten Polypeptids aufgrund einer Interaktion des anticodogenen Elements (a) mit einer durch die Ziel-Zelle exprimierten RNA, eine spezifische Prozessierung des RNA-Konstrukts erfolgt wodurch das Translationsinitiator-Element (d) aktiviert wird.

Weitere Aspekte der Vorliegenden Erfindung sind die Verwendung der hier beschrieben Verfahren und RNA-Konstrukte bei therapeutisch klinischen Anwendungen, bei Neoplasien, immunologischen Grunderkrankungen und metabolen Erkrankungen. Weiterhin sind die hier beschriebenen Verfahren und RNA-Konstrukte geeignet die notwendigen Komponenten zum Gene-editing Zelltyp spezifisch zu exprimieren und somit Germline- Erkrankungen zu behandeln.

Neben diesen typischen Anwendungen können auch adulte Stammzellen (iPS, multi, toti- oder pluripotent) von Säugern und deren abgeleitete Geweben als Zielzellen in vitro fungieren. Die aus diesen Zellen resultierenden Gewebe oder Einzelzellen können dann sowohl in vivo beim Menschen als auch in der Medikamenten-Entwicklung zum Einsatz kommen.

### Kurze Beschreibung der Abbildungen

- Abbildung 1:: Schematische Darstellung eines mRNA Moleküls. Sämtliche Abschnitte einer mRNA können für die Stabilität des Moleküls regulatorische Funktion aufweisen.
- Abbildung 2:: Schematische Darstellung einer Ausführungsform eines RNA-Konstrukts der vorliegenden Erfindung zur selektiven Expression von Zielsequenzen in spezifischen Zelltypen nach Organ- oder organismenweiter Einbringung des mRNA-Moleküls.

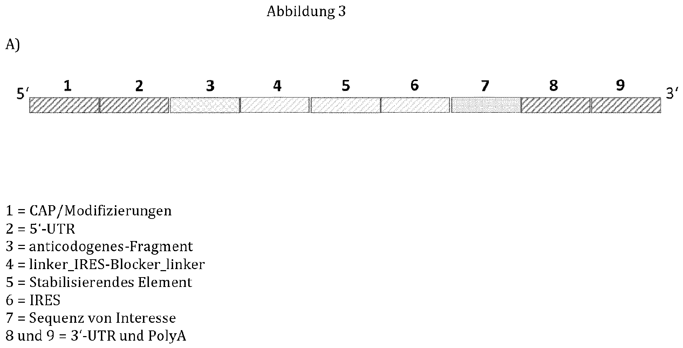

- Abbildung 3:: Schematische Darstellung einer weiteren Ausführungsform eines RNA-Konstrukts der vorliegenden Erfindung zur selektiven Expression von Zielsequenzen in spezifischen Zelltypen, wobei 1 = CAP/Modifizierungen, 2 = 5'-UTR, 3 = anticodogenes-Fragment, 4 = linker_IRES-Blocker_linker, 5 = Stabilisierendes Element, 6 = IHRES, 7 = Sequenz von Interesse, 8 und 9 = 3'-UTR und PolyA.
- Abbildung 4:: A) Schematische Darstellung einer Ausführungsform eines RNA-Konstrukts der vorliegenden Erfindung vor und nach der Prozessierung durch anticodogene Wechselwirkung zwischen Element 3 und zelltypspezifischer RNA. Das anticodogene Sequenzelement führt bei Bindung an Ziel-RNA zum Abbau der Motive 1-4 mittels etablierter Mechanismen. B) Berechnete Sekundärstruktur einer beispielhaften funktionalen IRES-Sequenz (6) aus HCV (HCV IRES 1b).

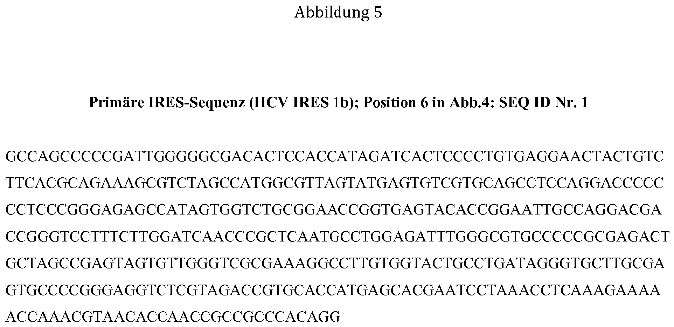

- Abbildung 5:: Primäre IRES- Nukleinsäuresequenz (HCV IRES 1b); Position 6 in Abb.4: SEQ ID Nr. 1. Die dazugehörige funktionale Sekundärstruktur ist in Abb. 4 aufgeführt.

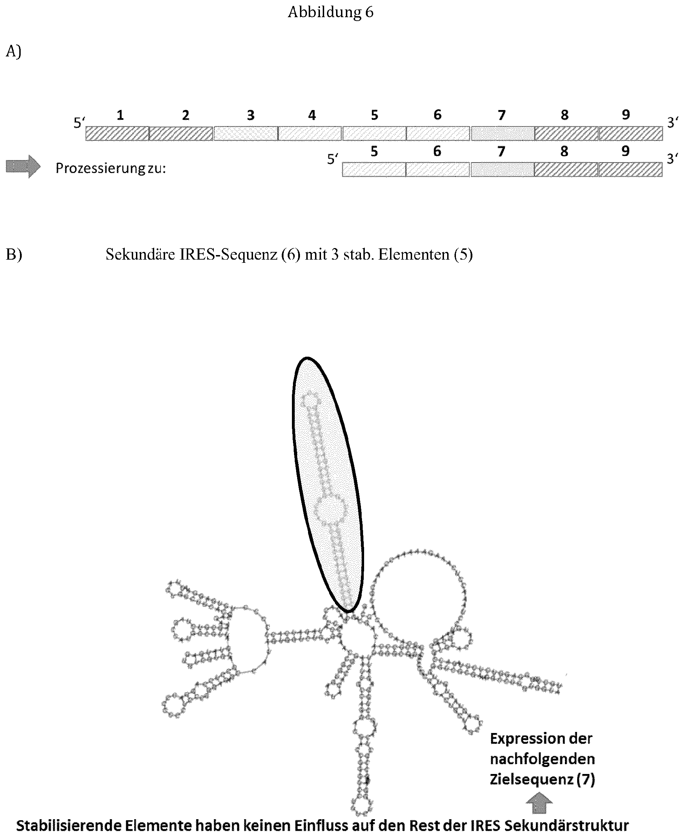

- Abbildung 6:: A) Schematische Darstellung einer Ausführungsform eines RNA-Konstrukts der vorliegenden Erfindung vor und nach der Prozessierung durch anticodogene Wechselwirkung zwischen Element 3 und zelltypspezifischer RNA. Das anticodogene Sequenzelement führt bei Bindung an Ziel-RNA zum Abbau der Motive 1-4 mittels etablierter Mechanismen. B) Schematische Darstellung der Sekundärstruktur einer beispielhaften IRES-Sequenz (6) aus HCV (HCV IRES 1b) mit 3 stab. Elementen (5).

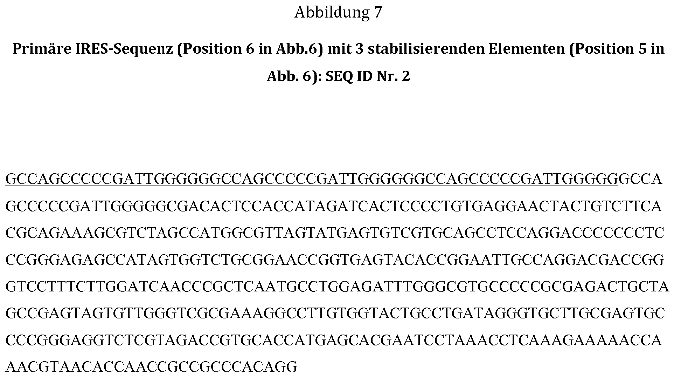

- Abbildung 7:: Primäre IRES-Sequenz (Position 6 in Abb.6) mit 3 stabilisierenden Elementen (Position 5 in Abb. 6): SEQ ID Nr. 2. Die dazugehörige funktionale Sekundärstruktur ist in Abb. 6 aufgeführt.

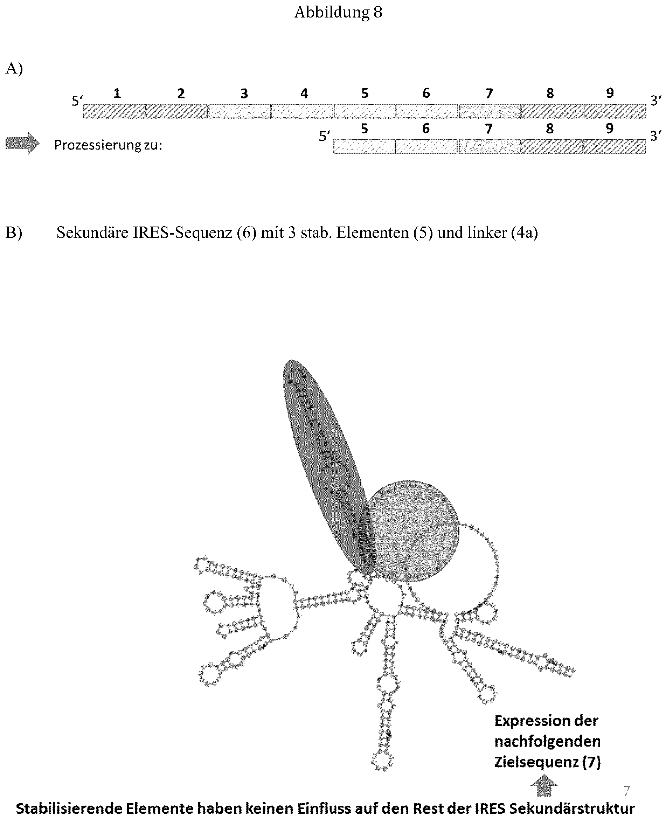

- Abbildung 8:: A) Schematische Darstellung einer Ausführungsform eines RNA-Konstrukts der vorliegenden Erfindung vor und nach der Prozessierung durch anticodogene Wechselwirkung zwischen Element 3 und zelltypspezifischer RNA. Das anticodogene Sequenzelement führt bei Bindung an Ziel-RNA zum Abbau der Motive 1-4 mittels etablierter Mechanismen. B) Schematische Darstellung der Sekundärstruktur einer beispielhaften IRES-Sequenz (6) aus HCV (HCV IRES 1b) mit 3 stab. Elementen (5) und Linker (4a).

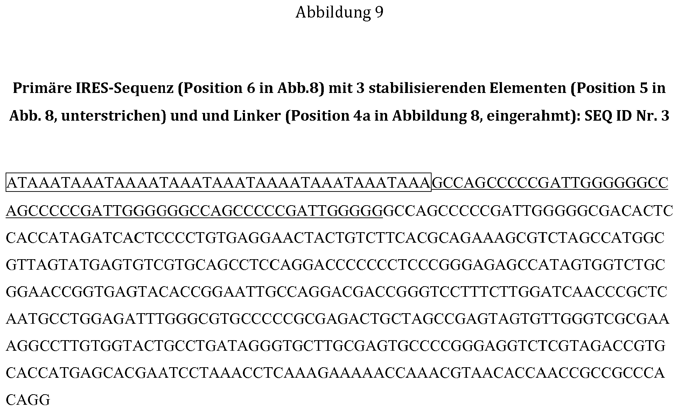

- Abbildung 9:: Primäre IRES-Sequenz (Position 6 in Abb.8) mit 3 stabilisierenden Elementen (Position 5 in Abb. 8, unterstrichen) und Linker (Position 4a in Abbildung 8, eingerahmt): SEQ ID Nr. 3. Die dazugehörige funktionale Sekundärstruktur ist in Abb. 8 aufgeführt.

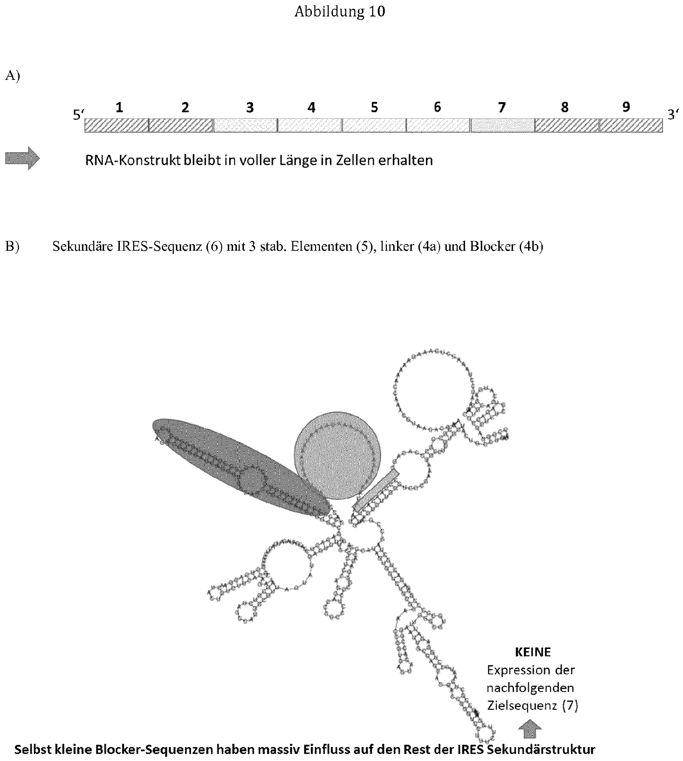

- Abbildung 10:: A) Schematische Darstellung einer Ausführungsform eines RNA-Konstrukts der vorliegenden Erfindung vor und nach der Prozessierung durch anticodogene Wechselwirkung zwischen Element 3 und zelltypspezifischer RNA. Das anticodogene Sequenzelement führt bei Bindung an Ziel-RNA zum Abbau der Motive 1-4 mittels etablierter Mechanismen. B) Schematische Darstellung der Sekundärstruktur einer beispielhaften IRES-Sequenz (6) aus HCV (HCV IRES 1b) mit 3 stab. Elementen (5), Linker (4a) und Blocker (4b).

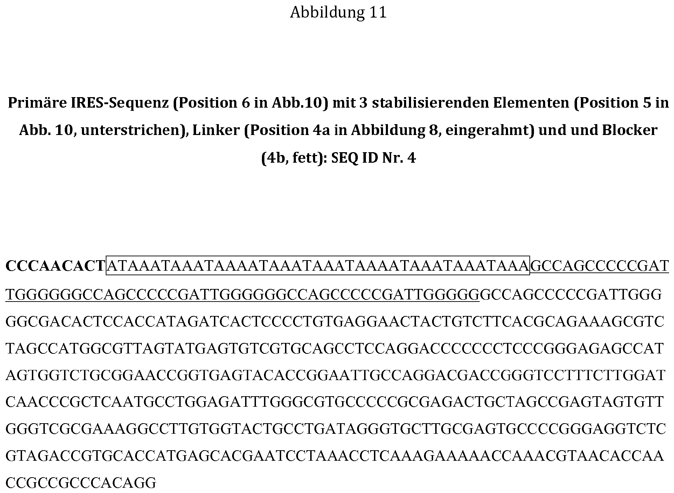

- Abbildung 11:: Primäre IRES-Sequenz (Position 6 in Abb.10) mit 3 stabilisierenden Elementen (Position 5 in Abb. 10, unterstrichen), Linker (Position 4a in Abbildung 8, eingerahmt) und und Blocker (4b, fett): SEQ ID Nr. 4. Die dazugehörige Sekundärstruktur ist in Abb. 10 aufgeführt.
- Abbildung 12:: Beispiel einer Nukleotidsequenz des funktionalen Systems zur selektiven Expression von Zielsequenzen in definierten Zelltypen.
- Abbildung 13:: Beispiel einer Nukleotidsequenz des funktionalen Systems mit alternativer antisense-Sequenz zur selektiven Expression von Zielsequenzen in definierten Zelltypen.
- Abbildung 14:: Beispiel einer Nukleotidsequenz des funktionalen Systems mit alternativer antisense-Sequenz und alternativer Blocker-Sequenz zur selektiven Expression von Zielsequenzen in definierten Zelltypen.
- Abbildung 15:: Mikroskopische Aufnahmen zur Zelltyp-abhängige, Blocker-IRES gesteuerte, selektive Expression von GFP.
- Abbildung 16:: Tabellen zur quantitativen Auswertung des funktionalen Systems zur selektiven Expression von Zielgenen.

Im Folgenden wird die Erfindung ohne Beschränkung des allgemeinen Erfindungsgedankens anhand von Ausführungsbeispielen unter Bezugnahme auf die Figuren näher erläutert.

Der Umfang der Erfindung wird durch die angehängten Ansprüche definiert.

### Detaillierte Beschreibung der Erfindung

Die grundlegende Struktur des RNA-Moleküls beinhaltet stabilisierende Strukturen, einen regulatorischen Bereich sowie einen Funktionsbereich, der für die Sequenz von Interesse kodiert. Die regulatorische Struktur fungiert als konstitutiver Inhibitor der Expression der Sequenz von Interesse. Die Inhibition wird nur aufgehoben, wenn spezifische Bindungen den selektiven Abbau des regulatorischen Abschnittes induzieren und so die Expression der Sequenz von Interesse ermöglichen.

Die Erfindung umfasst das Verfahren zum Aufbau geeigneter RNA-Sequenzen, mit deren Hilfe es möglich ist, nach Einbringung in beliebige Zelltypen, die Funktionalität einer Zielsequenz oder einer daraus entstehenden, auf Aminosäuren- basierenden Struktur nur in definierten Zelltypen zu induzieren und in allen anderen zu verhindern.

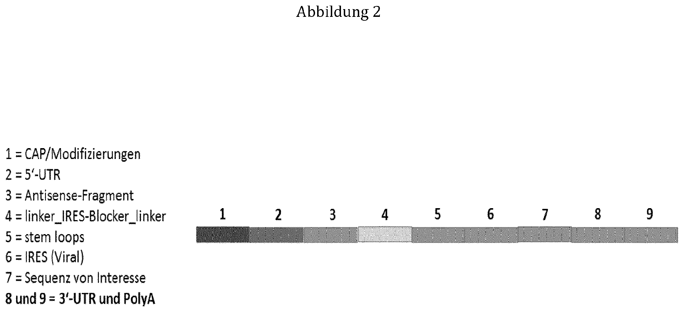

Zur Lösung des Problems einer definierten Expression von mRNA Molekülen in bestimmten Zelltypen nach Einbringung in den Gesamtorganismus oder Teilen davon, werden im Rahmen dieser Patentanmeldung unterschiedliche Sequenzmotive derart kombiniert, dass die funktionelle Translation der Sequenz von Interesse nur in gewünschten Zelltypen ermöglicht wird, während in anderen Zelltypen dieses unterbleibt. Durch Anpassung der Sequenzmotive kann das zelltypabhängige Wechselspiel zwischen Aktivierung und Inaktivierung angepasst oder auch komplett umgekehrt werden. Generell ist das System derart aufgebaut, dass vorgeschaltete Sequenzen im 5'-Bereich die Stabilität des Systems regulieren und gleichzeitig Sequenzmotive von Interesse im 3'-Bereich inhibieren bzw. allgemein regulieren. Inhibierte Sequenzen innerhalb des 3'-Bereichs sind im Besonderen internal ribosomal entry sites IRES (internal ribosomal entry site) -Sequenzen, die als sekundärer Translationsstart dienen können. Die Inhibition mittels Basenpaarung innerhalb des mRNA-Moleküls wird permanent aufrechterhalten solange bis die mRNA in geeigneten Zielzellen auf zelltypspezifische, endogene mRNA-Moleküle trifft, die mit Sequenzen im 5'-Bereich der eingebrachten mRNA antisense-aktive Basenpaarungen aufbaut und dadurch den Abbau des vorderen Bereichs der eingebrachten mRNA induziert. Hierdurch wird die Inhibition der IRES aufgehoben und die Expression der Zielsequenz ermöglicht. Im Detail erfolgt der Aufbau der zelltypspezifisch regulierbaren mRNA-Moleküle nach dem in Abb. 2 dargestellten Schema, welches nachfolgend näher erklärt ist. Hierbei können je nach Notwendigkeit einzelne Sequenzmotive auch ausgelassen, in ihrer Reihenfolge ausgetauscht, durch andere Motive ersetzt oder durch weitere Motive ergänzt werden.

Abb. 2. Zeigt eine schematische Darstellung eines mRNA Moleküls zur selektiven Expression von Zielsequenzen in spezifischen Zelltypen nach Organ- oder organismenweiter Einbringung des mRNA-Moleküls.
1) Optionale Verwendung einer CAP-Sequenz. Diese Sequenz kann ein natürliches 7-Methylguanosin (m7G)-Cap darstellen oder auch chemische oder andersartige Modifizierungen enthalten. Die CAP Sequenz dient der Regulation der Stabilität und Translationseffizienz der Primärsequenz. Für bestimmte Anwendungen, beispielsweise zur Entwicklung von RNA-Molekülen bzw. RNA-Fragmenten mit geringer Stabilität kann auch auf die CAP-Sequenz verzichtet werden.
2) Verwendung einer 5'-untranslatierten Sequenz. Diese Sequenz dient in erster Linie der Regulation der Stabilität (Halbwertszeit) der eingebrachten mRNA. Nukleotidsequenzen können entweder synthetischer Natur sein oder Sequenzen bekannter Gene entnommen werden. In Abhängigkeit der notwendigen Stabilität können in diesen Bereich zusätzliche Sequenzen einzeln oder in Kombination integriert werden, die Stabilität und Translationsrate regulieren. Beispiele wären hier G-quadruplexe (hohe mRNA Stabilität bei gleichzeitig niedriger Translationsrate) oder Bindestellen für Endonukleasen (Erniedrigung der mRNA Stabilität). Die Länge der 5'-untranslatierten Sequenz ist frei wählbar und kann je nach Notwendigkeit auch Null betragen.
3) Anschließend an die 5'-untranslatierte Sequenz startet die eigentliche Regulationskassette zur zelltyp-spezifischen Expression künstlich eingebrachter mRNA-Moleküle. Startpunkt ist eine Antisense- oder auch RNAi-aktive anticodogene Sequenz innerhalb der eingebrachten mRNA gegen ein spezifisch in einem gewünschten Zelltyp exprimiertes Gen. Wird das mRNA-Konstrukt in eine Zelle eingebracht, in der das zelltyp-spezifische Gen nicht transkribiert wird, erfolgt keine Wechselwirkung. Ist jedoch eine derartige Sense-mRNA vorhanden, erfolgt eine Basenpaarung zwischen Sense und Antisense-Strang, wodurch ein nachfolgender Abbau sowohl des sense als auch des antisense-Stranges induziert wird. Der Abbau selbst wird durch in allen Zellen konservierte Proteinkomplexe initiiert und durchgeführt, wobei im Besonderen der Enzymkomplex RISC (RNA-induced silencing complex) und die Ribonukleasen Dicer und Drosha eine wichtige Rolle spielen (Sontheimer EJ (2005). "Assembly and function of RNA silencing complexes". Nature Reviews Molecular Cell Biology. 6: 127-138). Abhängig von umgebenden Sequenzen kann der Abbau den gesamten eingebrachten mRNA-Strang umfassen oder aber durch zusätzliche Sequenzen eingeschränkt werden.

Zelltyp- oder auch organspezifische Proteinexpressionsmuster sind als Stand der Technik auf Basis moderner Transkriptom- bzw. Proteomanalysen für nahezu jeden Zelltyp oder Gewebe bekannt und können leicht als Grundlage für eine Vorauswahl dienen (siehe beispielhaft Ko et al. PNAS, 2013, 110:3095-3100 oder Whitfield et al. PNAS, 2003, 100:12319-12324).

Zur Identifizierung geeigneter anticodogener Elemente (3) mit bestmöglicher antisense Funktion können unterschiedliche Parameter berücksichtigt werden. Beispielhaft siehe: Elbashir SM, Harborth J, Lendeckel W, Yalcin A, Weber K, Tuschl T. Duplexes of 21-nucleotide RNAs mediate RNA interference in cultured mammalian cells. Nature. 2001 May 24;411(6836):494-8. Elbashir SM, Lendeckel W, Tuschl T. RNA interference is mediated by 21- and 22-nucleotide RNAs. Genes Dev. 2001 Jan 15;15(2):188-200. Reynolds A, Leake D, Boese Q, Scaringe S, Marshall WS, Khvorova A. Rational siRNA design for RNA interference. Nat Biotechnol. 2004 Mar;22(3):326-30.

Erfindungsgemäß sollten dabei folgende Parameter eine Rolle spielen:
- Anticodogene Sequenz möglichst 50 bis 100 nt downstream vom Startkodon
- Suche nach Sequenzmotiven AA(N₁₉)TT oder NA(N₂₁), oder NAR(N₁₇)YNN, mit N = beliebiges Nukleotid, R = Purin und Y = Pyrimidine
- Anticodogene Sequenz mit möglichst G+C Gehalt von 35-60%
- Vermeidung von vier oder mehr identischen Nukleotiden in Folge
- Vermeidung von Sequenzmotiven mit hoher Homologie zu anderen Genen

4) Anschließend zur antisense /RNAi aktiven Sequenz schließt sich eine Sequenz an, die in der Lage ist Basenpaarungen oder andersartige Wechselwirkungen mit der im 3'-Bereich des Konstruktes liegenden IRES- Sequenz einzugehen und so als IRES-Blocker zu fungieren. Da IRES-Sequenzen selbst ihre Funktion als CAP-unabhängiger Translationsinitiator ebenfalls hauptsächlich bis ausschließlich durch spezifisch aufgebaute Sekundärstrukturen auszuüben vermag, verändern die zu-sätzlichen Basenpaarungen zwischen IRES-Blocker und IRES-Sequenz diese derart, dass keine IRES-abhängige Translationsinitiation erfolgt solange der IRES-Blocker vorhanden ist. Die IRES-Blockierung ist bei voller Länge der eingebrachten mRNA der Fall. Da definiert induzierte Längenveränderungen der mRNA nur bei Bindung der spezifischen Antisense /RNAi Sequenz mit Sense-mRNA erfolgen, unterbleibt die IRES-Aktivität in allen Zellen, die nicht über eine passende Sense-mRNA verfügen. Anwesenheit dieser Sense-mRNA in spezifischen Zelltypen führt hingegen zur Antisense-Sense Bindung und dadurch zur Induktion des Abbaus der eingebrachten mRNA, wodurch auch die Sequenz des IRES-Blockers be-troffen wird und so ihre blockierende Wirkung auf die Sekundärstrukturbildung der IRES-Sequenz verliert. Die IRES Blocker Sequenz ist dadurch charakterisiert, dass sie i) anticodogene Abschnitte der IRES Sequenz beinhaltet, an ii) eine oder mehrere Sequenzabschnitte der IRES-Sequenz bindet, iii) zwischen den zur IRES-Sequenz anticodogenen Abschnitten Spacersequenzen aufweisen kann, die in Länge und Nukleotidzusammensetzung unter-schiedliche Zusammensetzungen aufweisen können, iv) bevorzugt an stem-Sequenzen oder loop-Sequenzen der IRES binden, um deren Sekundärstruktur zu verändern, v) dass die Se-quenzen eine variable Länge an zur IRES anti-codogenen Nukleotiden aufweisen ohne dabei typischerweise selbst eine antisense- oder RNAi Aktivität zu entfalten. Veränderungen in der Sekundärstruktur der IRES mittels des Blockers können hierbei nur minimal sein, um die Funktion der IRES effizient zu unterbinden und eine Rückfaltung der IRES-Sequenz bei Abbau des Blockers zu erlauben. Um eine bestmögliche Bindung von IRES-Blocker und IRES zu ermöglichen, kann die Sequenz des IRES-Blockers sowohl zu voranstehenden als auch nachfolgenden Sequenzmotiven von in der Länge variabel einstellbaren Linkersequenzen umgeben sein.

5) Um nach Bindung von Sense- und Antisense/RNAi mRNA-Motiven die komplette Degra-dation der eingebrachten mRNA auf spezifische Bereiche derselben einzuschränken, müssen mRNA-Sequenzmotive eingebaut werden, die aufgrund von Bindungspartnern oder ausgebildeter Sekundärstrukturen die Degradation unterbrechen. Derartige Sequenzmotive können Hairpin-loops oder stem-loops einschließen. Sie können in einfacher oder multipler Anordnung sowohl in 5'- als auch 3'-Richtung zur sense-antisense Paarung eingesetzt werden. Die Verwendung von Sequenzmotiven, die typischerweise durch Sekundärstrukturen oder Bindungspartner stabilisiert sind, bewirkt so in Kombination mit der spezifischen antisense/RNAi-Kassette in Zielzellen mit sense Paarung die Unterbrechung des mRNA-Abbaus und dadurch Stabilisierung der in 3'-Richtung zu dieser Sequenz liegenden RNA-Motiven. Hier übernehmen die stabilen Sekundärstruktu-ren dann die Funktion des CAP in Bezug auf Stabilisierung, nicht jedoch in Bezug auf Translationsinitiation. In Zellen, in denen keine sense-antisense Paarung erfolgt, bleibt die Gesamtlänge der mRNA erhalten und die loop-Strukturen verbleiben ohne signifikante Funktion. Stabile mRNA-Sekundärstrukturen können mit oder ohne Verbindungssequenzen (linker) mit benachbarten Sequenzmotiven verknüpft werden.

6) Da in Zielzellen die für die Translationsinitiation notwendige CAP-Sequenz abgespalten ist, muss hinter den stabilen mRNA-Sekundärstrukturen eine CAP unabhängige ribosomale Bindungsstelle in die Sequenz integriert werden, um eine Peptid- oder Proteinbildung zu initiieren. Hierzu wird eine IRES als Translationsinitiator fungierende Sequenz in 3'-Richtung eingebaut, die typischerweise aufgrund ihrer ausgebildeten Sekundärstruktur die direkte Bindung ribosomaler Untereinheiten ermöglicht. IRES Sequenzen können zellulärer, viraler oder auch synthetischer Natur sein. Aufgrund zur IRES-Sequenz passender IRES-Blockersequenzen im gleichen Volllängen mRNA-Konstrukt, die durch Basenpaarung die Ausbildung der für die Translationsinitiation notwendigen IRES-Sekundärstruktur unterbinden, ist die IRES-Sequenz in Nicht-Zielzellen und damit vorhandener Volllängen-mRNA inaktiv. In Zielzellen hingegen erfolgt durch sense-antisense/RNAi Wechselwirkung der Abbau der IRES-Blockersequenz wodurch durch energetisch getriebe-ne Prozesse die funktionsfähige IRES-Sekundärstruktur ausgebildet wird und für die Translation nachfolgender, kodierender Sequenzen fungieren kann. Die IRES-Sequenz kann mit oder ohne Linkersequenzen zu umgebenden Sequenzmotiven eingebaut werden.

7) 3' zur eingesetzten IRES Struktur können kodierende Sequenzen einge-bracht werden, die mittels Translation in Peptide umgeschrieben werden. Die Translation dieser Sequenzmotive erfolgt ausschließlich in Zellen in denen die IRES-Sequenz aktiv ist, also in Zielzellen, in denen zum Antisense / RNAi Motiv innerhalb der eingebrachten mRNA auch endogene sense-Sequenzen transkribiert werden. Zu translatierende RNA-Sequenzen können für bei-spielhaft therapeutisch aktive Peptide kodieren.

8) Nachfolgend zum definiert translatierbaren Leseraster sind in 3'-Richtung weitergehende Sequenzen notwendig, die wiederum die Stabilität sowohl der Volllängen-mRNA als auch der in Zielzellen eingekürzten mRNA gewährleisten. Diese Sequenzen umfassen sowohl den untranslatierten 3'-Bereich sowie den PolyA-Schwanz, der auch für nahezu jede endogene mRNA charakteristisch ist. In Abhängigkeit der Notwendigkeit, kann auf endogene oder auf synthetische Sequenzmotive zurückgegriffen werden. In Abhängigkeit der gewünschten Stabilität des mRNA-Konstruktes kann durch passende Wahl des Sequenzmotivs diese gezielt eingestellt werden. Von besonderer Wichtigkeit sind hierbei i) ausgebildete Sekundärstrukturen innerhalb des Motivs, ii) mögliche Bindungsstellen für stabilisierende / destabilisierende Faktoren sowie iii) Länge des PolyA-Schwanzes.

Basierend auf diesem System ist erstmalig die selektive einstellbare Expression von Zielsequenzen selbst bei Einbringung von mRNA-Molekülen in den gesamten Organismus möglich.

Die RNA-Konstrukte der vorliegenden Erfindung lassen sich auf unterschiedlichen Wegen in eine Zielzelle einbringen. Sie können per Transfektion, Membranfusion, Elektroporation oder als retroviraler Vektor in die Zelle gelangen. In der WO 02/44321 wird beispielsweise das Einbringen eines fremden Zielgens in eine Zelle offenbart.

In einer besonderen Ausführungsform der vorliegenden Erfindung führt die Interaktion des anticodogenen Elements (a) mit der durch die Ziel-Zelle exprimierten mRNA zu einem Abbau oder Inaktivierung des blockierenden Elements (b), so dass die Translation des Polypeptids von Interesse erfolgen kann. In einer weiteren Ausführungsform wird der Abbau der mRNA am stabilisierenden Element (c) gestoppt.

In weiteren bevorzugten Ausführungsformen der Erfindung weist das anticodogene Element (a) eines oder mehrere der folgenden Merkmale auf und hat folgende Funktion oder Funktionen nach Einbringen in die Zielzelle:
(i) Anlagerung an eine für die Ziel-Zelle spezifische RNA-Sequenz.
(ii) Bildung doppelsträngiger RNA-Hybride frei einstellbarer Länge
(iii) Induktion der zellulären Degradation des eingebrachten RNA-Konstruktes im Bereich des doppelsträngigen RNA-Hybrids.

In weiteren bevorzugten Ausführungsformen der Erfindung weist das blockierende Element (b) eines oder mehrere der folgenden Merkmale auf:
(i) die Funktion des Translationsinitiator-Elementes wird unterdrückt.
(ii) das blockierende Element unter geeigneten Bedingungen abgebaut oder inaktiviert werden kann.
(iii) die unterdrückende Funktion auf das Translationsinitiator-Element nach Inaktivierung oder Abbau des blockierenden Elementes reversibel ist.
(iv) das blockierende Element seine Funktion mittels Basenpaarung in oder in der Nähe der Sequenz des Translationsinitiator-Elements ausübt und dadurch die Sekundärstruktur desselben ändert.
(v) das blockierende Element seine Funktion dadurch ausübt, dass es andere Moleküle dazu bringt das Translationsinitiator-Element direkt oder indirekt zu inhibieren.

In weiteren bevorzugten Ausführungsformen der Erfindung weist das stabilisierende Element (c) eines oder mehrere der folgenden Merkmale auf:
(i) Nukleotidsequenz, die die Degradation der RNA in 3'-Richtung durch Exo- und/oder Endonukleasen unterbindet.
(ii) Nukleotidsequenz dadurch gekennzeichnet, dass sie stabilisierende Sekundärstrukturen, wie beispielsweise stem-loops, ausbildet.
(iii) Nukleotidsequenz, dadurch gekennzeichnet, dass stabilisierende Elemente wie beispielsweise Peptide oder Proteine an die RNA binden.
(iv) Nukleotidsequenz, dadurch gekennzeichnet, dass Moleküle an die RNA binden und eine chemische, stabilisierende Modifizierung des RNA 5'-Endes vornehmen.

In weiteren bevorzugten Ausführungsformen der Erfindung weist das Translationsinitiator-Element (d) eines oder mehrere der folgenden Merkmale aufweist:
(i) Zelltypabhängige, selektive Inhibierbarkeit der Funktion der Translationsinitiation
(ii) Funktionalität ausschließlich bei Degradation oder Inaktivierung der Bestandteile der regulatorischen Kassette (Elemente a und b)
(iii) Funktionalität ausschließlich in Zielzellen, während in allen anderen Zelltypen das Translationsinitiator-Element inaktiv ist.
(iv) Induktion der Translation unabhängig von der Cap-Struktur im 5'-Bereich des Ausgangs-RNA-Konstrukts.
(v) Induktion der Translation mittels direkter oder indirekter Bindung ribosomaler Untereinheiten oder kompletter Ribosomen (z.B. IRES Sequenzen).
(vi) Induktion der Translation durch Ausbildung oder Freigabe spezifischer Sekundärstrukturen nach Wegfall der Inhibition durch das blockierende Element
(vii) Induktion der Translation durch Anlagerung spezifischer Moleküle oder chemische Modifikation nach Wegfall der Inhibition durch das blockierende Element

In besonders bevorzugten Ausführungsformen der vorliegenden Erfindung weist das RNA-Konstrukt eine Kombination aus anticodogener- und IRES-abhängiger Expressionsregulation auf.

Der Abbau des blockierenden Elements kann beispielsweise über das System des antisense- oder siRNA-abhängigen mRNA Abbaus erfolgen. Dem aus der Literatur als "RNA-Interferenz" (RNAi) bekannten Phänomen liegt zugrunde, dass kurze RNA-Moleküle (siRNA, small interfering RNA) in der Zelle mit Messenger-RNA (mRNA) in Wechselwirkung treten können (Literatur: Fire A., Xu S., Montgomery M.K., Kostas S.A., Driver S.E., Mello C.C., Nature Feb 19, 1998; 391 (6669):744-745). Über einen komplexen Mechanismus, der von Enzymen gesteuert wird (beispielsweise über den DICER- und RISC-Komplex), kommt es zu einem Abbau (einer Degradation) der mRNA. Neben der siRNA wurden weitere kleine RNA Spezies entdeckt, beispielsweise die "micro- RNAs" (miRNA) oder "short hairpin RNAs" (shRNA), die ebenfalls mittels verwandter Mechanismen eine Proteinexpression inhibieren können.

Die durch den Abbau und/oder Inaktivierung des blockierenden Elements eingeleitete Translationsinitiation des RNA-Konstrukts in der Zielzelle ist ein komplexer Prozess, der die konzertierte Interaktion zahlreicher Faktoren beinhaltet (Pain, V. M., 1996, Eur. J. Biochem. 236, 747-771). Für die meisten mRNAs besteht der erste Schritt in der Rekrutierung ribosomaler 40S Untereinheiten zur RNA an oder in der Nähe ihres 5'-Endes (Fig. 1). Die Assoziierung von 40S an die mRNA wird von dem Cap-bindenden Komplex elF4F sehr erleichtert. Faktor elF4F besteht aus drei Untereinheiten: der RNA-Helicase eIF4A, dem Cap-Bindeprotein elF4E und dem Multiadaptorprotein elF4 G, das als Rahmen für die Proteine in dem Komplex wirkt und Bindestellen für elF4E, elF4a, elF3 und ein Poly(A)-bindendes Protein aufweist.

Die Infektion von Zellen mit einer Vielzahl von RNA Viren führt zur selektiven Inhibition der Translation von mRNA des Wirts jedoch nicht des Virus. Zum Beispiel führt die Infektion von Zellen mit dem Poliovirus, einem zytoplasmatischen RNA Virus, zur Modifizierung mehrerer Translationsinitiationsfaktoren. Inbesondere die Proteolyse beider Formen von elF4G, elF4Gl und elF4Gll (Gradi et al. 1998, Proc. Nat. Acad. Sci. USA 95, 11089-11094), durch viruskodierte Proteasen führt zur Inhibition der Translation der meisten zellulären mRNAs mit Cap. Dagegen wird die Translation polioviraler mRNAs, die eine spezifische, 450 Nukleotide umfassende Sequenz als IRES im 5'-UTR enthalten, welche 40S Untereinheiten in Abwesenheit von intaktem elF4F rekrutieren kann, nicht inhibiert (Jang et al. 1988 J. Virol. 62, 2363-2643). IRES Elemente wurden in picornaviraler, flaviviraler, pestiviraler, retroviraler, lentiviraler, insektenviraler RNA und in tierischer zellulärer RNA gefunden. Übersichten zu bekannten IRES-Sequenzmotiven stellen einen Stand der Wissenschaft dar (als Übersicht dargestellt auf: http://www.iresite.org (Stand 1.2.2017). IRES enthaltende tierische mRNAs können ribosomale Untereinheiten sowohl über ihr 5'-Cap-Ende als auch über ihre IRES-Elemente rekrutieren. Hierdurch ist eine Translation auch unter Bedingungen möglich, unter denen die Capabhängige Translation reduziert ist, wie z. B. während einer viralen Infektion, während der G2/M-Phase des Zellzyklus, Apoptose- oder Stressbedingungen (Johannes et al. (1999), Proc. Natl. Acad. Sci. USA 96, 13118-13123; Cornelis et al. (2000), molecular Cell 5, 597-605; Pyronnet et al. (2000), molecular Cell 5, 607-616; Stein et al., 1998, Mol. and Cell. Biol. 18, 3112-3119; Holcik et al., 2000, Oncogene 19, 4174-4177; Stoneley et al., 2000, mol. and Cell. Biol. 20, 1162-1169). Bis zu 3% der zellulärer mRNAs von Tieren werden bei verminderter Konzentration des Cap-bindenden Komplexes elF4F translatiert (Johannes et al., 1999).

Für die RNA-Konstrukte der vorliegenden Erfindung können eine Vielzahl unterschiedlicher IRES-Elemente als Translationsinitiator-Element verwendet werden. Beispielsweise werden im Stand der Technik IRES-Elemente beschrieben, die zur Cap-unabhängigen Expression fremder Gene in linearer multicistronischer mRNA in tierischen Zellen benutzt werden (US 6 060 273; US 6 114 146; US 5 358 856; US 6 096 505; US 171 821; US 5 766 903), in Pflanzenzellen (WO 98/54342) und, allgemein, in eukaryotischen Zellen (US 171 821; US 5 766 903; US 5 925 565; US 6 114 146).

Beispielsweise bleibt die Sekundärstruktur des IRES-Elements unbeeinflusst und damit funktionell und wird hier nur durch geeignete Wahl des blockierenden Elementes (b) verändert (= inaktiviert).

In weiteren bevorzugten Ausführungsformen der Erfindung weist das RNA-Konstrukt eines oder mehrere der folgenden optionalen Merkmale auf:
(i) 5'-Cap Sequenz, um die Stabilität des mRNA-Gesamtkonstruktes zu erhöhen
(ii) 5'-untranslatierter Bereich, um die Stabilität des mRNA-Gesamtkonstruktes regulieren zu können und die 5'-Cap induzierte Translation zu unterdrücken (z.B. Einbau von Quadruplex-Sequenzen)
(iii) 3'-untranslatierter Bereich, um die Stabilität des mRNA-Gesamtkonstruktes sowie des prozessierten mRNA-Teilfragmentes in Zielzellen zu erhöhen.
(iv) Poly-A Bereich, um die Stabilität des mRNA-Gesamtkonstruktes sowie des prozessierten mRNA-Teilfragmentes in Zielzellen zu erhöhen.
(v) Sämtliche Elemente des mRNA-Gesamtkonstruktes aus Ansprüchen 1 bis 8 können linker-Sequenzen beliebiger Länge innerhalb des Elementes selbst oder aber zwischen den einzelnen Elementen enthalten. Linkersequenzen können funktionslos sein und der reinen räumlichen Trennung der einzelnen Elemente dienen, oder zusätzliche Funktionen mit in das System integrieren.

### Ausführungsbeispiele:

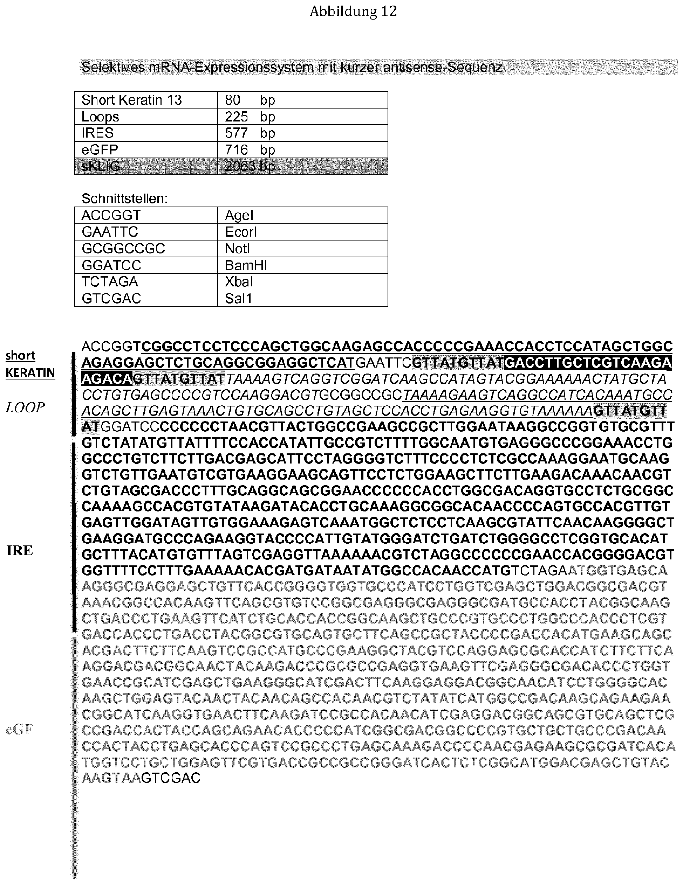

### 1.) Nukleotidsequenz des funktionalen Systems zur selektiven Expression von Zielsequenzen in definierten Zelltypen (Abbildung 12).

Mittels molekularbiologischer Standardprozeduren PCR, Ligation und Transformation) wurde eine kurze Sequenz des Keratin13 Gens (oben markiert mit **short KERATIN**) des humanen Genoms als antisense mit einer sogenannten *LOOP*-Sequenz verbunden. Die *LOOP*-Sequenz beinhaltet **Linker**-Sequenzen, die der Flexibilität des Konstruktes dienen, eine **Blocker**-Sequenz, sowie Nukleotidsequenzen, die stabile Sekundärstrukturen ausbilden (*LOOPs*)*.* Während die **Blocker**-Sequenz in der Lage ist mit Sequenzabschnitten der **IRES** durch Basenpaarung zu interagieren und dadurch deren Sekundärstruktur zu verändern, bewirken die Sekundärstrukturen der *LOOPs* eine Unterbrechung von Exo-Nukleaseaktivität (siehe Chapman et al. eLife, 2014,3:e01892). Nachfolgende Sequenzabschnitte stellen die **IRES** des Encephalomyocarditis Virus (Bochkov and Palmenberg, BioTechniques, 2006, 41:283-292) sowie die kodierende Sequenz des grün fluoreszierenden Proteins **(eGFP)** dar. Die **IRES** erlaubt bei Wegfall der **Blocker**-Sequenz die Translation nachfolgender Sequenzen - in diesem Fall des **eGFP,** welches hier aus Gründen der leichten Nachweisbarkeit gewählt wurde. Wegfall der **Blocker**-Sequenz erfolgt nur, wenn für die Antisense-Sequenz (**short KERATIN**) im gewählten Zelltyp eine komplementäre Sense-Sequenz zur Bindung vorliegt.

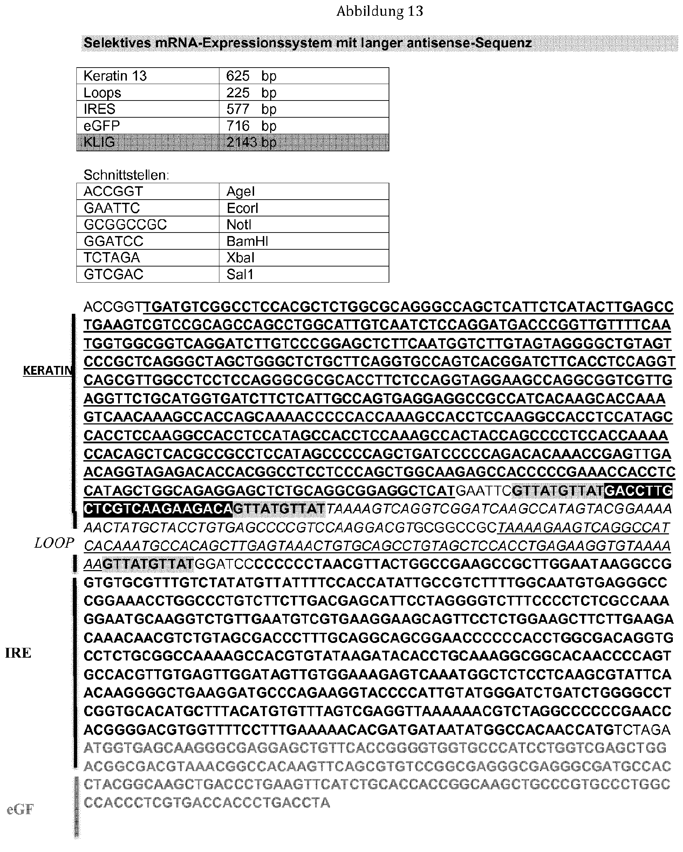

### 2.) Nukleotidsequenz des funktionalen Systems mit alternativer antisense-Sequenz zur selektiven Expression von Zielsequenzen in definierten Zelltypen (Abbildung 13).

Mittels molekularbiologischer Standardprozeduren PCR, Ligation und Transformation) wurde eine lange Sequenz des Keratin13 Gens (oben markiert mit **KERATIN)** des humanen Genoms als antisense mit einer sogenannten *LOOP*-Sequenz verbunden. Die *LOOP*-Sequenz beinhaltet **Linker**-Sequenzen, die der Flexibilität des Konstruktes dienen, eine Blocker-Sequenz, sowie Nukleotidsequenzen, die stabile Sekundärstrukturen ausbilden (*LOOPs*). Während die **Blocker**-Sequenz in der Lage ist mit Sequenzabschnitten der **IRES** durch Basenpaarung zu interagieren und dadurch deren Sekundärstruktur zu verändern, bewirken die Sekundärstrukturen der *LOOPs* eine Unterbrechung von Exo-Nukleaseaktivität (siehe Chapman et al. eLife, 2014,3:e01892). Nachfolgende Sequenzabschnitte stellen die **IRES** des Encephalomyocarditis Virus (Bochkov and Palmenberg, BioTechniques, 2006, 41:283-292) sowie die kodierende Sequenz des grün fluoreszierenden Proteins **(eGFP)** dar. Die **IRES** erlaubt bei Wegfall der **Blocker**-Sequenz die Translation nachfolgender Sequenzen - in diesem Fall des **eGFP**, welches hier aus Gründen der leichten Nachweisbarkeit gewählt wurde. Wegfall der **Blocker-**Sequenz erfolgt nur, wenn für die Antisense-Sequenz **(KERATIN**) im gewählten Zelltyp eine komplementäre Sense-Sequenz zur Bindung vorliegt.

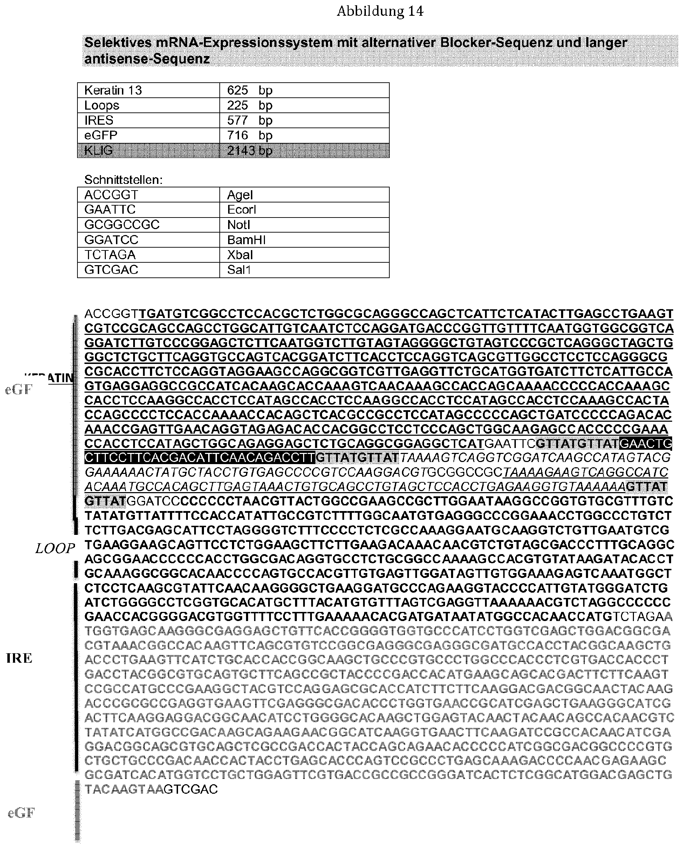

### 3.) Nukleotidsequenz des funktionalen Systems mit alternativer antisense-Sequenz und alternativer Blocker-Sequenz zur selektiven Expression von Zielsequenzen in definierten Zelltypen (Abbildung 14).

Mittels molekularbiologischer Standardprozeduren PCR, Ligation und Transformation) wurde eine lange Sequenz des Keratin13 Gens (oben markiert mit **KERATIN)** des humanen Genoms als antisense mit einer sogenannten *LOOP*-Sequenz verbunden. Die *LOOP*-Sequenz beinhaltet **Linker**-Sequenzen, die der Flexibilität des Konstruktes dienen, eine **Blocker**-Sequenz, sowie Nukleotidsequenzen, die stabile Sekundärstrukturen ausbilden (*LOOPs*). Während die **Blocker**-Sequenz in der Lage ist mit Sequenzabschnitten der **IRES** durch Basenpaarung zu interagieren und dadurch deren Sekundärstruktur zu verändern, bewirken die Sekundärstrukturen der *LOOPs* eine Unterbrechung von Exo-Nukleaseaktivität (siehe Chapman et al. eLife, 2014,3:e01892). Nachfolgende Sequenzabschnitte stellen die **IRES** des Encephalomyocarditis Virus (Bochkov and Palmenberg, BioTechniques, 2006, 41:283-292) sowie die kodierende Sequenz des grün fluoreszierenden Proteins **(eGFP)** dar. Die **IRES** erlaubt bei Wegfall der **Blocker**-Sequenz die Translation nachfolgender Sequenzen - in diesem Fall des **eGFP,** welches hier aus Gründen der leichten Nachweisbarkeit gewählt wurde. Wegfall der **Blocker-**Sequenz erfolgt nur, wenn für die Antisense-Sequenz (**KERAT**IN) im gewählten Zelltyp eine komplementäre Sense-Sequenz zur Bindung vorliegt.

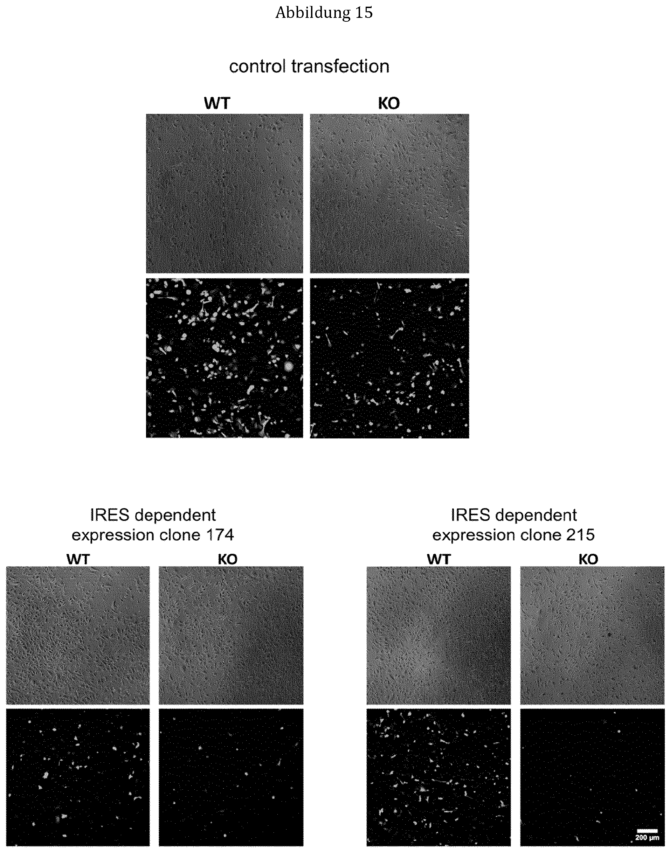

### 4.) Zelltyp-abhängige, Blocker-IRES gesteuerte, selektive Expression von GFP (Abbildung 15).

Epidermalzellen der Maus (Keratinozyten) mit (WT) und ohne (knockout Keratin13 Mutante=KO) Keratin13 mRNA wurden mit GFP-Expressionsplasmiden als Positivkontrolle behandelt. Nach 24 h wurden die Zellen licht- (grau) sowie fluoreszenzmikroskopisch (grün) untersucht. Als Resultat der Übertragung ist in beiden Zelllinien eine gleichmäßig hohe Expression von GFP zu erkennen.

Durch Verwendung der in Abb. 1 (Klon 174) und 2 (Klon 215) generierten Konstrukte erfolgt die Expression von GFP stark bevorzugt in solchen Zellen, die Keratin13 mRNA exprimieren und kaum in solchen, die deletiert für das Keratin13 Gen sind. Begründet ist dieser Effekt dadurch, dass in Abwesenheit von Keratin13 mRNA die Blockersequenz innerhalb des generierten mRNA-Konstruktes mit der IRES-Sequenz wechselwirkt und durch Veränderung der IRES-Sekundärstruktur deren Funktion und damit GFP-Expression unterdrückt. Im Gegensatz dazu wird in Anwesenheit der Keratin13-mRNA (WT) das generierte mRNA-Konstrukt durch sense-antisense Wechselwirkung bis zu den loops abgebaut, wodurch die Sequenz des Blockers verschwindet. Hierdurch wird die Funktion der IRES freigegeben und die Expression des GFP erfolgt.

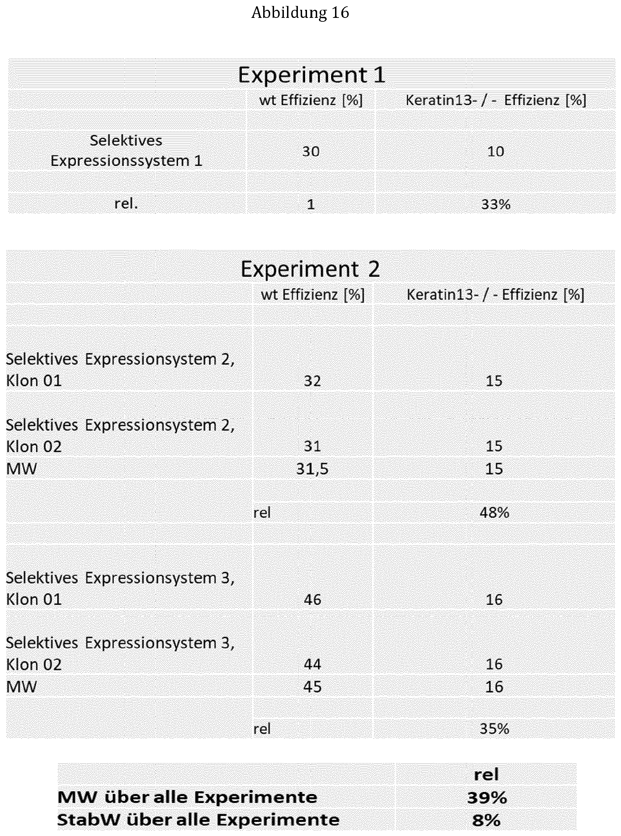

### 5.) Quantitative Auswertung des funktionalen Systems zur selektiven Expression von Zielgenen (Abbildung 16).

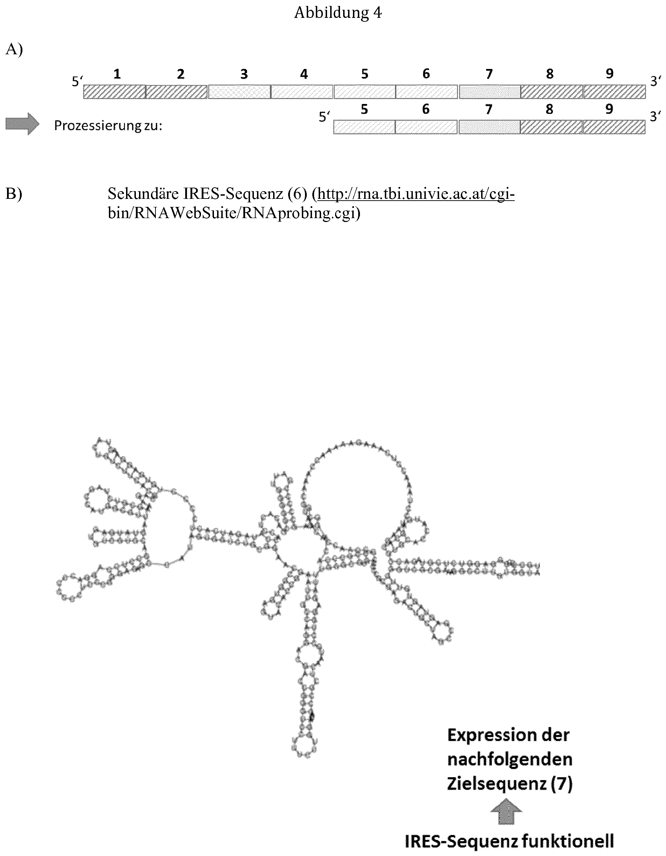

Wildtyp und Keratin13 Mutantenzellen wurden wie in Abb. 4 beschrieben mit den unterschiedlichen funktionalen Systemen beladen. Nach 24 Stunden wurde in allen Zellen die Expressionsstärke des gewählten Zielproteins GFP mittels Durchflusszytometrie gemessen. Pro Messzahl wurden jeweils mindestens 5000 Zellen ausgewertet. Alle Unterschiede zwischen WT und Keratin13-/- Mutante sind hoch-signifikant.

## Patentansprüche

1. Verfahren zur zielgerichteten Translation eines Polypeptids von Interesse in einer eukaryotischen Ziel-Zelle, **gekennzeichnet durch** das Einbringen eines RNA-Konstrukts in die Ziel-Zelle, wobei das RNA-Konstrukt in 5' nach 3' Richtung zumindest folgende Elemente aufweist:
(a) ein anticodogenes Element (a), das zumindest zu einem Abschnitt einer mRNA eines durch die Ziel-Zelle exprimierten Gens komplementär ist;
(b) ein blockierendes Element (b), das mindestens mit einem Abschnitt des 3' liegenden Translationsinitiator-Elements (d) wechselwirkt;
(c) ein stabilisierendes Element (c);
(d) ein Translationsinitiator-Element (d), das eine internal ribosomal entry site (IRES) - Sequenz aufweist;
(e) eine für das Polypeptid von Interesse kodierende Sequenz (e),
wobei in einer eukaryotischen Nicht-Ziel-Zelle die Translation des Polypeptids von Interesse durch Basenpaarung des blockierende Elements (b) mit dem Translationsinitiator Element (d) verhindert wird und in einer eukaryotischen Ziel-Zelle die Translation des kodierten Polypeptids aufgrund einer durch die Interaktion des anticodogenen Elements mit der durch die Ziel-Zelle exprimierten mRNA induzierten Prozessierung des RNA-Konstrukts erfolgt.

2. Verfahren nach Anspruch 1, wobei die Interaktion des anticodogenen Elements (a) mit der durch die Ziel-Zelle exprimierten mRNA zu einem Abbau oder Inaktivierung des blockierenden Elements (b) führt, so dass die Translation des Polypeptids von Interesse erfolgen kann.

3. Verfahren nach Anspruch 2, wobei der Abbau der mRNA am stabilisierenden Element (c) gestoppt wird.

4. Verfahren nach einem oder mehreren der Ansprüche 1 bis 3, **dadurch gekennzeichnet, dass** das anticodogene Element (a) eines oder mehrere der folgenden Merkmale aufweist:
(i) Anlagerung an eine für die Ziel-Zelle spezifische RNA-Sequenz.
(ii) Bildung doppelsträngiger RNA-Hybride frei einstellbarer Länge
(iii) Induktion der zellulären Degradation des eingebrachten RNA-Konstruktes im Bereich des doppelsträngigen RNA-Hybrids.

5. Verfahren nach einem oder mehreren der Ansprüche 1 bis 4, **dadurch gekennzeichnet, dass** das blockierende Element (b) eines oder mehrere der folgenden Merkmale aufweist:
(i) die Funktion des Translationsinitiator-Elementes (d) unterdrückt
(ii) das blockierende Element (b) unter geeigneten Bedingungen abgebaut oder inaktiviert werden kann.
(iii) die unterdrückende Funktion auf das Translationsinitiator-Element (d) nach Inaktivierung oder Abbau des blockierenden Elementes reversibel ist.
(iv) das blockierende Element (b) seine Funktion mittels Basenpaarung in oder in der Nähe der Sequenz des Translationsinitiator-Elements (d) ausübt und dadurch die Sekundärstruktur desselben ändert
(v) das blockierende Element (b) seine Funktion dadurch ausübt, dass es andere Moleküle dazu bringt das Translationsinitiator-Element (d) direkt oder indirekt zu inhibieren.

6. Verfahren nach einem oder mehreren der Ansprüche 1 bis 5, **dadurch gekennzeichnet, dass** das stabilisierende Element (c) eines oder mehrere der folgenden Merkmale aufweist:
(i) Nukleotidsequenz, die die Degradation der RNA durch Exo- und/oder Endonukleasen in 3'-Richtung unterbindet.
(ii) Nukleotidsequenz **dadurch gekennzeichnet, dass** sie stabilisierende Sekundärstrukturen, wie beispielsweise stem-loops, ausbildet.
(iii) Nukleotidsequenz, **dadurch gekennzeichnet, dass** stabilisierende Elemente wie beispielsweise Peptide oder Proteine an die RNA binden.
(iv) Nukleotidsequenz, **dadurch gekennzeichnet, dass** Moleküle an die RNA binden und eine chemische, stabilisierende Modifizierung des RNA 5'-Endes vornehmen.

7. Verfahren nach einem oder mehreren der Ansprüche 1 bis 6, **dadurch gekennzeichnet, dass** das Translationsinitiator-Element (d) eines oder mehrere der folgenden Merkmale aufweist:
(i) Zelltypabhängige, selektive Inhibierbarkeit der Funktion der Translationsinitiation
(ii) Funktionalität ausschließlich bei Degradation oder Inaktivierung der Bestandteile der regulatorischen Kassette (Elemente a und b)
(iii) Funktionalität ausschließlich in Zielzellen, während in allen anderen Zelltypen das Translationsinitiator-Element inaktiv ist.
(iv) Induktion der Translation unabhängig von der Cap-Struktur im 5'-Bereich des Ausgangs-RNA-Konstrukts.
(v) Induktion der Translation mittels direkter oder indirekter Bindung ribosomaler Untereinheiten oder kompletter Ribosomen.
(vi) Induktion der Translation durch Ausbildung spezifischer Sekundärstrukturen nach Wegfall der Inhibition durch das blockierende Element
(vii) Induktion der Translation durch Anlagerung spezifischer Moleküle oder chemische Modifikation nach Wegfall der Inhibition durch das blockierende Element

8. Verfahren nach einem oder mehreren der Ansprüche 1 bis 7, **dadurch gekennzeichnet, dass** das RNA-Konstrukt eines oder mehrere der folgenden optionalen Merkmale aufweist:
(i) 5'-Cap Sequenz, um die Stabilität des mRNA-Gesamtkonstruktes zu erhöhen
(ii) 5'-untranslatierter Bereich, um die Stabilität des mRNA-Gesamtkonstruktes regulieren zu können und die 5'-Cap induzierte Translation zu unterdrücken (z.B. Einbau von Quadruplex-Sequenzen)
(iii) 3'-untranslatierter Bereich, um die Stabilität des mRNA-Gesamtkonstruktes sowie des prozessierten mRNA-Teilfragmentes in Zielzellen zu regulieren.
(iv) Poly-A Bereich, um die Stabilität des mRNA-Gesamtkonstruktes sowie des prozessierten mRNA-Teilfragmentes in Zielzellen zu regulieren.
(v) Sämtliche Elemente des mRNA-Gesamtkonstruktes aus Ansprüchen 1 bis 8 können linker-Sequenzen beliebiger Länge innerhalb des Elementes selbst oder aber zwischen den einzelnen Elementen enthalten. Linkersequenzen können funktionslos sein und der reinen räumlichen Trennung der einzelnen Elemente dienen, oder zusätzliche Funktionen mit in das System integrieren.

9. RNA-Konstrukt zur zielgerichteten Translation eines Polypeptids von Interesse in einer eukaryotischen Ziel-Zelle, wobei das RNA-Konstrukt in 5' nach 3' Richtung zumindest folgende Elemente aufweist:
(a) ein anticodogenes Element (a), das zumindest zu einem Abschnitt einer RNA eines durch die Ziel-Zelle exprimierten DNA-Abschnitts komplementär ist oder den Ziel-Zell-spezifischen Abbau induziert;
(b) ein blockierendes Element (b), das zumindest zu einem Abschnitt des 3' liegenden Translationsinitiator-Elements (d) komplementär ist oder dieses anderweitig inhibiert;
(c) ein stabilisierendes Element (c), das den Ziel-Zell-spezifischen Abbau der RNA auf einen Teilbereich derselben beschränkt;
(d) ein Translationsinitiator-Element (d), das unabhängig vom 5'-Cap funktioniert und nur in Zielzellen aktiv ist; wobei das Translationsinitiator-Element (d) eine internal ribosomal entry site (IRES) - Sequenz aufweist;
(e) eine für das Polypeptid von Interesse kodierende Sequenz (e);
wobei in einer eukaryotischen Nicht-Ziel-Zelle die Translation des Polypeptids von Interesse durch Basenpaarung des blockierende Elements (b) mit dem Translationsinitiator-Element (d) verhindert wird und in einer eukaryotischen Ziel-Zelle die Translation des kodierten Polypeptids aufgrund einer Interaktion des anticodogenen Elements (a) mit einer durch die Ziel-Zelle exprimierten RNA, eine spezifische Prozessierung des RNA-Konstrukts erfolgt wodurch das Translationsinitiator-Element aktiviert wird.

10. RNA-Konstrukt nach Anspruch 9, **gekennzeichnet durch** ein oder mehrere der folgenden Merkmale:
(i) das anticodogene Element (a) weist ein oder mehrere der Merkmale des Anspruchs 4 auf;
(ii) das blockierende Element (b) weist ein oder mehrere der Merkmale des Anspruchs 5 auf;
(iii) das stabilisierende Element (c) weist ein oder mehrere der Merkmale des Anspruchs 6 auf;
(iv) das Translationsinitiator-Element (d) weist ein oder mehrere der Merkmale des Anspruchs 7 auf; und/oder
(v) das mRNA Konstrukt weist ein oder mehrere der Merkmale des Anspruchs 8 auf.

## Claims

1. Method for targeted translation of a polypeptide of interest in a eukaryotic target cell, **characterized by** the introduction of an RNA construct into the target cell, the RNA construct having at least the following elements in the 5' to 3' direction:
(a) an anticodogenic element (a) that is complementary to at least a portion of an mRNA of a gene expressed by the target cell;
(b) a blocking element (b) that interacts with at least a portion of the translation initiator element (d) at the 3' position;
(c) a stabilizing element (c);
(d) a translation initiator element (d) that has an internal ribosomal entry site (IRES) sequence;
(e) a sequence (e) encoding the polypeptide of interest,
the translation of the polypeptide of interest being prevented in a eukaryotic non-target cell by base pairing of the blocking element (b) with the translation initiator element (d), and the translation of the encoded polypeptide taking place in a eukaryotic target cell due to processing of the RNA construct induced by the interaction of the anticodogenic element with the mRNA expressed by the target cell.

2. Method according to claim 1, wherein the interaction of the anticodogenic element (a) with the mRNA expressed by the target cell results in degradation or inactivation of the blocking element (b), so that translation of the polypeptide of interest can take place.

3. Method according to claim 2, wherein the degradation of the mRNA is stopped at the stabilizing element (c).

4. Method according to one or more of claims 1 to 3, **characterized in that** the anticodogenic element (a) has one or more of the following features:
(i) attachment to an RNA sequence specific to the target cell.
(ii) formation of double-stranded RNA hybrids of freely adjustable length
(iii) induction of cellular degradation of the introduced RNA construct in the region of the double-stranded RNA hybrid.

5. Method according to one or more of claims 1 to 4, **characterized in that** the blocking element (b) has one or more of the following features:
(i) the function of the translation initiator element (d) is suppressed.
(ii) the blocking element (b) can be degraded or inactivated under suitable conditions.
(iii) the suppressive function on the translation initiator element (d) is reversible after inactivation or degradation of the blocking element.
(iv) the blocking element (b) performs its function by base pairing in or near the sequence of the translation initiator element (d) and thereby alters the secondary structure thereof.
(v) the blocking element (b) performs its function by causing other molecules to directly or indirectly inhibit the translation initiator element (d).

6. Method according to one or more of claims 1 to 5, **characterized in that** the stabilizing element (c) has one or more of the following features:
(i) nucleotide sequence that prevents degradation of RNA by exo- and/or endonucleases in the 3' direction.
(ii) nucleotide sequence, **characterized in that** it forms stabilizing secondary structures, such as stem loops.
(iii) nucleotide sequence, **characterized in that** stabilizing elements such as peptides or proteins bind to the RNA.
(iv) nucleotide sequence, **characterized in that** molecules bind to the RNA and perform a chemical, stabilizing modification of the RNA 5' end.

7. Method according to one or more of claims 1 to 6, **characterized in that** the translation initiator element (d) has one or more of the following features:
(i) cell type-dependent, selective inhibitability of the translation initiation function
(ii) functionality only upon degradation or inactivation of the components of the regulatory cassette (elements a and b)
(iii) functionality exclusively in target cells, while the translation initiator element is inactive in all other cell types.
(iv) induction of translation independent of the cap structure in the 5' region of the initial RNA construct.
(v) induction of translation by direct or indirect binding of ribosomal subunits or complete ribosomes.
(vi) induction of translation by formation of specific secondary structures after cessation of inhibition by the blocking element
(vii) induction of translation by attachment of specific molecules or chemical modification after cessation of inhibition by the blocking element

8. Method according to one or more of claims 1 to 7, **characterized in that** the RNA construct has one or more of the following optional features:
(i) 5' cap sequence in order to increase the stability of the entire mRNA construct
(ii) 5' untranslated region in order to be able to regulate the stability of the entire mRNA construct and to suppress the 5' cap induced translation (e.g. incorporation of quadruplex sequences)
(iii) 3' untranslated region in order to regulate the stability of the entire mRNA construct as well as the processed mRNA fragment in target cells.
(iv) poly(A) region in order to regulate the stability of the entire mRNA construct as well as the processed mRNA fragment in target cells.
(v) all elements of the entire mRNA construct from claims 1 to 8 may contain linker sequences of any length within the element itself or between the individual elements. Linker sequences can be functionless and used purely to spatially separate the individual elements, or they can integrate additional functions into the system.

9. RNA construct for targeted translation of a polypeptide of interest in a eukaryotic target cell, wherein the RNA construct has at least the following elements in the 5' to 3' direction:
(a) an anticodogenic element (a) that is complementary to at least a portion of an RNA of a DNA portion expressed by the target cell, or induces target cell-specific degradation;
(b) a blocking element (b) that is complementary to or otherwise inhibits at least a portion of the translation initiator element (d) at the 3' position;
(c) a stabilizing element (c) that restricts the target cell-specific degradation of the RNA to a section thereof;
(d) a translation initiator element (d) that functions independently of the 5' cap and is only active in target cells; wherein the translation initiator element (d) has an internal ribosomal entry site (IRES) sequence;
(e) a sequence (e) encoding the polypeptide of interest;
wherein the translation of the polypeptide of interest is prevented in a eukaryotic non-target cell by base pairing of the blocking element (b) with the translation initiator element (d), and the translation of the encoded polypeptide in a eukaryotic target cell due to an interaction of the anticodogenic element (a) with an RNA expressed by the target cell, a specific processing of the RNA construct takes place, whereby the translation initiator element is activated.

10. RNA construct according to claim 9, **characterized by** one or more of the following features:
(i) the anticodogenic element (a) has one or more of the features of claim 4;
(ii) the blocking element (b) has one or more of the features of claim 5;
(iii) the stabilizing element (c) has one or more of the features of claim 6;
(iv) the translation initiator element (d) has one or more of the features of claim 7; and/or
(v) the mRNA construct has one or more of the features of claim 8.

## Revendications

1. Procédé de traduction ciblée d'un polypeptide d'intérêt dans une cellule cible eucaryote, **caractérisé par** l'introduction d'une construction d'ARN dans la cellule cible, dans lequel la construction d'ARN présente au moins les éléments suivants en direction 5' à 3' :
(a) un élément anticodogénique (a) qui est complémentaire d'au moins une section d'un ARNm d'un gène exprimé par la cellule cible ;
(b) un élément bloquant (b) qui interagit avec au moins une section de l'élément initiateur de traduction (d) situé en 3' ;
(c) un élément stabilisateur (c) ;
(d) un élément initiateur de traduction (d) qui présente une séquence de site d'entrée interne des ribosomes (IRES) ;
(e) une séquence (e) codant pour le polypeptide d'intérêt,
dans lequel, dans une cellule non cible eucaryote, la traduction du polypeptide d'intérêt est empêchée par appariement de bases de l'élément bloquant (b) avec l'élément initiateur de traduction (d) et, dans une cellule cible eucaryote, la traduction du polypeptide codé est effectuée en raison d'un traitement de la construction d'ARN induit par l'interaction de l'élément anticodogénique avec l'ARNm exprimé par la cellule cible.

2. Procédé selon la revendication 1, dans lequel l'interaction de l'élément anticodogénique (a) avec l'ARNm exprimé par la cellule cible conduit à une dissolution ou à une inactivation de l'élément bloquant (b), de sorte que la traduction du polypeptide d'intérêt peut être effectuée.

3. Procédé selon la revendication 2, dans lequel la dissolution de l'ARNm est arrêtée au niveau de l'élément stabilisant (c).

4. Procédé selon une ou plusieurs des revendications 1 à 3,
**caractérisé en ce que** l'élément anticodogénique (a) présente une ou plusieurs des caractéristiques suivantes :
(i) addition à une séquence d'ARN spécifique à la cellule cible.
(ii) formation d'hybrides d'ARN double brin de longueur librement ajustable
(iii) induction de la dégradation cellulaire de la construction d'ARN introduite dans la région de l'hybride d'ARN double brin.

5. Procédé selon une ou plusieurs des revendications 1 à 4,
**caractérisé en ce que** l'élément bloquant (b) présente une ou plusieurs des caractéristiques suivantes :
(i) suppression de la fonction de l'élément initiateur de traduction (d).
(ii) l'élément bloquant (b) peut être dissous ou inactivé dans des conditions appropriées.
(iii) la fonction de suppression sur l'élément initiateur de traduction (d) est réversible après inactivation ou dissolution de l'élément bloquant.
(iv) l'élément bloquant (b) exerce sa fonction au moyen d'un appariement de bases dans ou à proximité de la séquence de l'élément initiateur de traduction (d), modifiant ainsi la structure secondaire de celui-ci
(v) l'élément bloquant (b) exerce sa fonction en amenant d'autres molécules pour inhiber directement ou indirectement l'élément initiateur de traduction (d).

6. Procédé selon une ou plusieurs des revendications 1 à 5,
**caractérisé en ce que** l'élément stabilisant (c) présente une ou plusieurs des caractéristiques suivantes :
(i) séquence de nucléotides qui empêche la dégradation de l'ARN par des exonucléases et/ou des endonucléases dans la direction 3'.
(ii) séquence de nucléotides, **caractérisé en ce qu'**elle réalise des structures secondaires stabilisantes, telles que des tiges-boucles par exemple.
(iii) séquence de nucléotides, **caractérisé en ce que** des éléments stabilisants, tels que des peptides ou des protéines par exemple, se lient à l'ARN.
(iv) séquence de nucléotides, **caractérisé en ce que** des molécules se lient à l'ARN et pratiquent une modification chimique stabilisatrice de l'extrémité 5' de l'ARN.

7. Procédé selon une ou plusieurs des revendications 1 à 6,
**caractérisé en ce que** l'élément initiateur de traduction (d) présente une ou plusieurs des caractéristiques suivantes :
(i) capacité d'inhibition sélective de la fonction de l'initiation de traduction dépendant du type de cellule
(ii) fonctionnalité uniquement en cas de dégradation ou d'inactivation des constituants de la cassette régulatrice (éléments a et b)
(iii) fonctionnalité uniquement dans des cellules cibles, alors que dans tous les autres types de cellules, l'élément initiateur de traduction est inactif.
(iv) induction de la traduction indépendamment de la structure CAP dans la région 5' de la construction de l'ARN de départ.
(v) induction de la traduction au moyen d'une liaison directe ou indirecte de sous-unités ribosomiques ou de ribosomes complets.
(vi) induction de la traduction par une formation de structures secondaires spécifiques après une levée de l'inhibition par l'élément bloquant
(vii) induction de la traduction par une addition de molécules spécifiques ou modification chimique après une levée de l'inhibition par l'élément bloquant

8. Procédé selon une ou plusieurs des revendications 1 à 7,
**caractérisé en ce que** la construction d'ARN présente une ou plusieurs des éventuelles caractéristiques suivantes :
(i) séquence 5'-CAP pour augmenter la stabilité de la construction globale de l'ARNm
(ii) région non traduite en 5' pour pouvoir réguler la stabilité de la construction globale de l'ARNm et supprimer la traduction induite en 5'-CAP (par exemple, insertion de séquences quadruplex)
(iii) région non traduite en 3' pour réguler la stabilité de la construction globale de l'ARNm ainsi que du fragment partiel d'ARNm traité dans les cellules cibles.
(iv) région poly A pour réguler la stabilité de la construction globale de l'ARNm ainsi que du fragment partiel d'ARNm traité dans les cellules cibles.
(v) tous les éléments de la construction globale d'ARNm des revendications 1 à 8 peuvent contenir des séquences de liaison de n'importe quelle longueur à l'intérieur de l'élément en lui-même ou entre les éléments individuels. Les séquences de liaison peuvent être sans fonction et servir à la simple séparation spatiale des éléments individuels, ou intégrer des fonctions supplémentaires dans le système.

9. Construction d'ARN pour la traduction ciblée d'un polypeptide d'intérêt dans une cellule cible eucaryote, dans laquelle la construction d'ARN présente au moins les éléments suivants en direction 5' à 3' :
(a) un élément anticodogénique (a) qui est complémentaire d'au moins une section d'un ARNm d'une section d'ADN exprimée par la cellule cible ou qui induit la dissolution spécifique à la cellule cible ;
(b) un élément bloquant (b) qui est complémentaire d'au moins une section de l'élément initiateur de traduction (d) situé en 3', ou bien l'inhibe ;
(c) un élément stabilisant (c) qui limite la dissolution spécifique à la cellule cible de l'ARN à une région partielle de celui-ci ;
(d) un élément initiateur de traduction (d) qui fonctionne indépendamment de 5'-CAP et qui est actif uniquement dans des cellules cibles ; dans laquelle l'élément initiateur de traduction (d) présente une séquence de site d'entrée interne des ribosomes (IRES) ;
(e) une séquence (e) codant pour le polypeptide d'intérêt ;
dans laquelle, dans une cellule non cible eucaryote, la traduction du polypeptide d'intérêt est empêchée par appariement de bases de l'élément bloquant (b) avec l'élément initiateur de traduction (d) et, dans une cellule cible eucaryote, la traduction du polypeptide codé effectue, en raison d'une interaction de l'élément anticodogénique (a) avec un ARN exprimé par la cellule cible, un traitement spécifique de la construction d'ARN, activant ainsi l'élément initiateur de traduction.

10. Construction d'ARN selon la revendication 9, **caractérisée par** une ou plusieurs des caractéristiques suivantes :
(i) l'élément anticodogénique (a) présente une ou plusieurs des caractéristiques de la revendication 4 ;
(ii) l'élément bloquant (b) présente une ou plusieurs des caractéristiques de la revendication 5 ;
(iii) l'élément stabilisateur (c) présente une ou plusieurs des caractéristiques de la revendication 6 ;
(iv) l'élément initiateur de traduction (d) présente une ou plusieurs des caractéristiques de la revendication 7 ; et/ou
(v) la construction d'ARNm présente une ou plusieurs des caractéristiques de la revendication 8.
